# EUROPEAN PATENT SPECIFICATION

(11) **EP 1 896 592 B1**
(45) Date of publication and mention of the grant of the patent: **17.03.2010**
(21) Application number: 06745162.5
(22) Date of filing: 21.06.2006
(51) Int. Cl.: C12N 15/82, A01H 5/00

(54) **METHODS FOR GENERATING PLANTS EXHIBITING ALTERED PLASMODESMATAL CONDUCTANCE**
VERFAHREN ZUR ERZEUGUNG VON Pflanzen mit VERÄNDERTEr PLASMODESMATA-LEITFähigkeit
PROCÉDÉS DESTINÉS À GÉNÉRER DES PLANTES MANIFESTANT UNE CONDUCTANCE PLASMODESMATALE MODIFIÉE

(30) Priority: 21.06.2005 US 692240 P
(43) Date of publication of application: 12.03.2008
(73) Proprietor: Ramot at Tel Aviv University Ltd., 69975 Tel Aviv (IL)
(72) Inventor: EPEL, Bernard L., 46309 Herzlia (IL); SAGI, Guy, 43601 RaAnana (IL)
(74) Representative: Machtalère, Georges
(86) International application number: PCT/IL2006/000723
(87) International publication number: WO 2006/137064

(56) References cited:
- WO-A-97/20470
- WO-A-99/67404
- WO-A2-94/04693
- WO-A2-99/58653
- BOCCA SILVIA N ET AL: "Molecular cloning and characterization of the enzyme UDP-glucose: Protein transglucosylase from potato" PLANT PHYSIOLOGY AND BIOCHEMISTRY (PARIS), vol. 37, no. 11, November 1999 (1999-11), pages 809-819, XP002405944 ISSN: 0981-9428
- DATABASE EMBL [Online] 20 March 1997 (1997-03-20), "Zea mays golgi associated protein se-wap41 mRNA, complete cds." XP002405948 retrieved from EBI accession no. EM_PRO:U89897 Database accession no. U89897
- EPEL B L ET AL: "A 41 KDA PROTEIN ISOLATED FROM MAIZE MESOCOTYL CELL WALLS IMMUNOLOCALIZED TO PLASMODESMATA" PROTOPLASMA, SPRINGER VERLAG, VIENNA, AT, vol. 191, 1996, pages 70-78, XP002071949 ISSN: 0033-183X cited in the application
- EPEL B L ET AL: "Plant virus movement protein dynamics probed with a GFP-protein fusion" GENE, ELSEVIER, AMSTERDAM, NL, vol. 173, no. 1, 1996, pages 75-79, XP004042856 ISSN: 0378-1119
- DELGADO I J ET AL: "Cloning and characterization of AtRGP1. A reversibly autoglycosylated arabidopsis protein implicated in cell wall biosynthesis" PLANT PHYSIOLOGY, AMERICAN SOCIETY OF PLANT PHYSIOLOGISTS, ROCKVILLE, MD, US, vol. 116, no. 4, April 1998 (1998-04), pages 1339-1349, XP002118093 ISSN: 0032-0889
- DHUGGA K S ET AL: "A reversibly glycosylated polypeptide (RGP1) possibly involved in plant cell wall synthesis: purification, gene cloning, and trans-Golgi localization" PROCEEDINGS OF THE NATIONAL ACADEMY OF SCIENCES OF USA, NATIONAL ACADEMY OF SCIENCE, WASHINGTON, DC, US, vol. 94, no. 14, 8 July 1997 (1997-07-08), pages 7679-7684, XP002118094 ISSN: 0027-8424 cited in the application
- ROBERTS A G ET AL: "Plasmodesmata and the control of symplastic transport." PLANT CELL AND ENVIRONMENT, vol. 26, no. 1, January 2003 (2003-01), pages 103-124, XP002405945 ISSN: 0140-7791
- SAGI GUY ET AL: "Class 1 reversibly glycosylated polypeptides are plasmodesmal-associated proteins delivered to plasmodesmata via the Golgi apparatus" PLANT CELL, vol. 17, no. 6, June 2005 (2005-06), pages 1788-1800, XP002405946 ISSN: 1040-4651

## Description

The present invention relates to a method of decreasing the ability of plasmodesmata to transport molecules or viruses in a plant tissue according to claim 1, a method of generating plant tissue resistant according to claim 2.

The present invention relates to plants exhibiting altered plasmodesmatal conductance and to constructs and methods utilizable for generating such plants.

Plasmodesmata (Pd) (plasmodesma singular) are specialized membranous tunnels thai interconnect neighboring plant cells and facilitate direct cytoplasmic cell-to-cell transport through the cell walls. Transport through Pd mediates many processes, among them, information transfer to allow the coordination of development, movement of photosynthesis products from mature to developing tissues, responses to pathogen attacks, systemic gene silencing, etc. (reviewed by Ding et al., 1999; Zambryski and Crawford, 2000; Haywood et al., 2002; Heinlein, 2002; Heinlein and Epel, 2004). In addition to their normal function, Pd are exploited by viruses and viroids for cell-to-cell movement and the long distance spread of infection (reviewed by Ding et al., 1999; Beachy and Heinlein, 2000; Citovsky and Zambryski, 2000; Gafni and Epel, 2002; Heinlein and Epel, 2004).

In higher plant, Pd may be mono-coaxial tunnels, termed simple Pd, or they may be branched, forming a network of interconnected coaxial tunnels, (Ehlers and Kollmann, 2001). In young (sink) leaves, most Pd are simple, whereas in mature (source) leaves there are both simple and branched Pd (Oparka et al., 1999). The outer boundary of a plasmodesma is delineated by a membrane continuous with the plasma membranes (PMs) of the two adjoining cells. Concentric with the outer plasma membrane is a second membrane termed desmotubule that is derived from and continuous with the ER membranes of the two cells. Between the concentric membranes is a sleeve that interconnects the cytoplasm of the neighboring cells. High-resolution electron micrographs reveal that within this sleeve are particles, which have been variously interpreted as being embedded in the PM (Ding et al., 1992) and/or in the desmotubule (Botha et al., 1993) or to be present entirely within the cytoplasmic sleeve (Overall, 1999).

Contrary to the structural analysis, knowledge regarding the biochemical composition of Pd is still fragmentary, largely due to the difficulty in isolating pure Pd without sub-cellular membranes contaminants. The present inventors have previously disclosed a procedure for isolating cell walls containing embedded Pd (Epel et al., 1996). Wall associated proteins (WAPs) extracted from a wall fraction prepared from the mesocotyls of etiolated maize seedlings were separated by SDS-PAGE and an antiserum was prepared against a band of approximately 41kDa that was highly enriched in this fraction. This antiserum, termed S-41, immunolocalized mainly to Pd and the Golgi apparatus (Epel et al., 1996). The 4kDa protein was shown to be released from the wall fraction by 3M NaCl or by pH 11, but not by 2% Triton X-100, indicating that this protein is a peripheral membrane protein (Epel et al., 1996).

While reducing the present invention to practice, the present inventors have cloned and sequenced the gene that encodes for the salt extractable 41kDa protein (termed herein SE-WAP41) and have shown that it is a member of the Reversibly Glycosylated Polypeptides (RGPs) protein family. While further reducing the present invention, the present inventors have also shown that class 1 RPGs (also referred to herein as C1RPG) target the plasmodesmata and alter its conductance. Thus, as is further described herein, the present inventors propose that RGPs can be utilized in regulating spread of plant viruses, altering plant morphology as well as other commercially important applications.

The method of decreasing the ability of plasmodesmata to transport molecules or viruses in a plant tissue is defined in claim 1 of the present invention, the method of generating plant tissue resistant is defined in claim 2 of the present invention.

According to one aspect of the present invention there is provided a plant cell comprising a heterologous polynucleotide sequence being capable of directing overexpression of a reversibly glycosylated polypeptide or a functional portion thereof.

A genetically modified plant tissue resistant to spread of a plant virus comprising cells over expressing a reversibly glycosylated polypeptide or a functional portion thereof is provided

Disclosed is provided a method of altering plasmodesmatal conductance in plant tissue comprising regulating an expression level of a reversibly glycosylated polypeptide or a functional portion thereof in cells of the plant tissue thereby altering plasmodesmatal conductance in the plant tissue.

According to still another aspect of the present invention there is provided a method of generating plant tissue resistant to spread of a plant virus comprising overexpressing in cells of the plant tissue a reversibly glycosylated polypeptide (pfam 03 214) thereby generating the plant tissue resistant to spread of the plant virus.

According to further features in preferred embodiments of the invention described below, the heterologous polynucleotide is a transcriptional regulator and the reversibly glycosylated polypeptide is an endogenous reversibly glycosylated polypeptide (pfam 03 214)

According to still further features in the described preferred embodiments the reversibly glycosylated polypeptide is encoded by a sequence selected from the group consisting of SEQ ID NOs: 8, 13 and 25-28.

According to still further features in the described preferred embodiments the functional portion is amino acids 270-end of SEQ ID NOS: 19-24.

According to still further features in the described preferred embodiments the plant cell/tissue forms a part of a plant tissue or a whole plant.

Also disclosed is a plant cell/tissue further comprising an additional heterologous polynucleotide encodes a molecule selected from the group consisting of a reporter molecule, an antiviral molecule, a viral moiety, a herbicide resistance molecule, a biotic or abiotic stress tolerance molecule, a pharmaceutical molecule, a growth inducing molecule and a growth inhibiting molecule.

According to still further features in the described preferred embodiments altering plasmodesmatal conductance is decreasing plasmodesmatal conductance.

According to still further features in the described preferred embodiments decreasing plasmodesmatal conductance is effected by upregulating the expression level of said reversibly glycosylated polypeptide in the cells.

According to still further features in the described preferred embodiments upregulating is effected by expressing in the plant tissue a heterologous polynucleotide encoding said reversibly glycosylated polypeptide fused to a bulking polypeptide.

According to still further features the plant is selected from the group consisting of tomato, potato, cucumber, rice, maize, wheat, oats, barley, rye, soybean, canola, rape and cotton.

According to still further features the over expressing is effected by introducing into the cells a nucleic acid construct encoding said reversibly glycosylated polypeptide or the functional portion thereof.

Also disclosed is a genetically modified plant exhibiting a dwarf phenotype comprising cells over expressing a reversibly glycosylated polypeptide.

The genetically modified plant is selected from the group consisting of tomato, potato, cucumber, rice, maize, wheat, oats, barley, rye, soybean, canola, rape and cotton.

Also disclosed is a method of generating a plant having a dwarf phenotype comprising overexpressing in cells of a plant a reversibly glycosylated polypeptide thereby generating the plant having the dwarf phenotype.

Also disclosed is a method of preventing spread of a viral construct to a specific tissue of a plant comprising overexpressing in the specific tissue of a plant a reversibly glycosylated polypeptide thereby preventing spread of the viral construct to the specific tissue of a plant.

Also disclosed is a viral construct is a viral expression construct expressing a molecule.

Said molecule is selected from the group consisting of a reporter molecule, an antiviral molecule, a viral moiety, a herbicide resistance molecule, a biotic or abiotic stress tolerance molecule, a pharmaceutical molecule, a growth inducing molecule and a growth inhibiting molecule.

According to still further features the overexpressing in the specific tissue of the plant the reversibly glycosylated polypeptide is effected such that the molecule is capable of accumulating in the specific plant tissue.

Also disclosed is an expression construct comprising a first polynucleotide sequence encoding at least a functional portion of a reversibly glycosylated polypeptide.

The expression construct further comprises a promoter sequence capable of directing expression of the first polynucleotide sequence in plant cells. said expression construct further comprises a second polynucleotide sequence being in translational fusion with the first polynucleotide sequence.

According to still further features the second polynucleotide sequence encodes a polypeptide selected from the group consisting of GUS, tdTomato (reviewed in Wei Wen Su 2005) and a GUS-tdTomato fusion.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

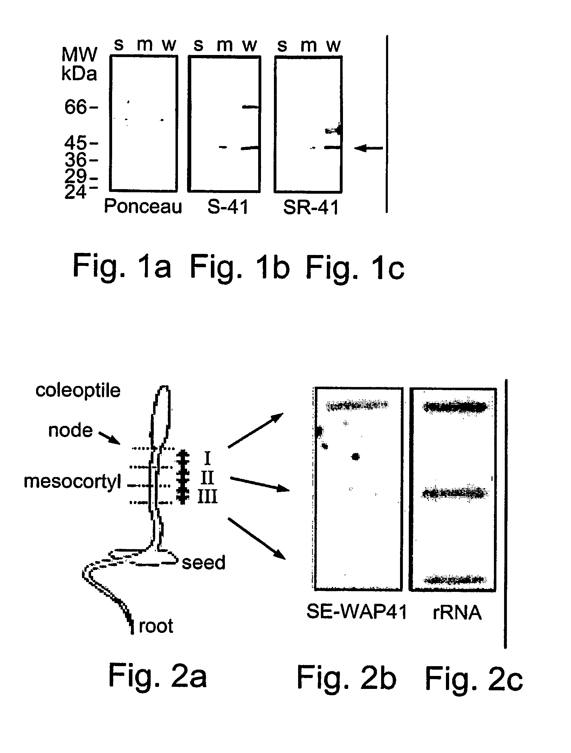

FIGs. 1a-c are a Western blot analysis illustrating SE-WAP41 enrichment in wall fraction of *Zea mays* L. cv. Jublee. Proteins extracted from the soluble (s), membranal (m) and wall (w) fractions were separated by SDS-PAGE (6µg/lane), electro-blotted to nitrocellulose and stained with Ponceau (left), reacted with S-41 antiserum (center) or with SR-41 antiserum (right). Arrow indicates 41kDa.
FIGs. 2a-c depict slot blot analysis of SE-WAP41 mRNA (Figure 2b) from mesocotyl sections of 5 day old etiolated maize seedlings (Figure 2a). RNA samples were prepared from upper mesocotyl segments: section I contains mesocotyl meristem and region of most rapid cell growth; section II also contains zone of elongation; section III contains mature non-dividing and non elongating cells. The ribosomal 26S rRNA probe served as a loading control (Figure 2c).

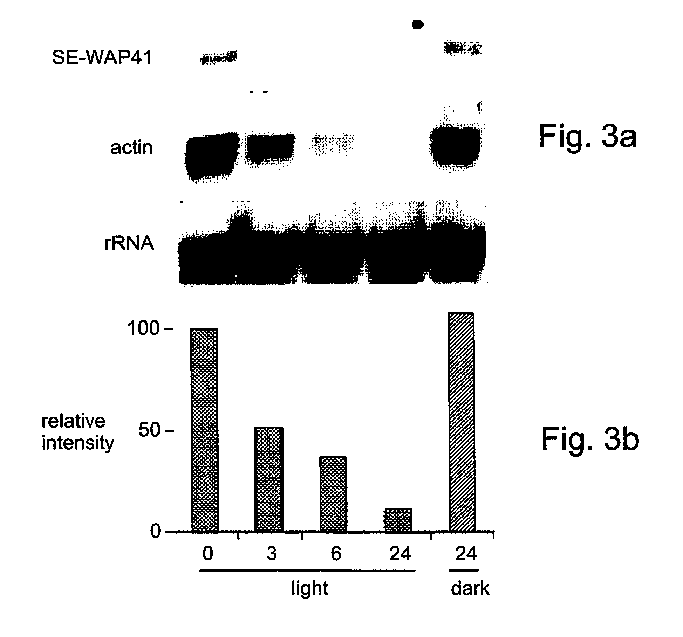

FIGs. 3a-b illustrate SE-WAP41 mRNA levels of etiolated maize seedlings exposed to light. Figure 3a - five days old etiolated maize seedlings were exposed to white light for 3, 6 and 24 hours or kept in the dark for 24 hours. A blot of total RNA extracted from the upper 1cm of mesocotyls (see Figure 2c) was hybridized to cDNAs of SE-WAP41, actin and 26S- rRNA. The ribosomal 26S rRNA probe served as a loading control. Actin mRNA levels decrease is indicative of cell division cessation. Figure 3b is a graph depicting relative SE-WAP41 mRNA levels. Values were calculated relative to 26S-rRNA intensity in each lane.

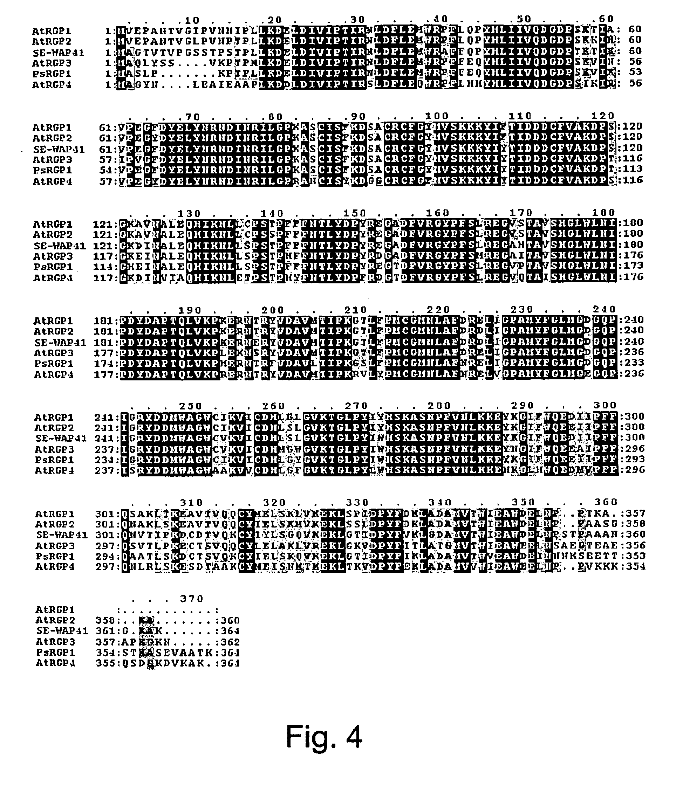

FIG. 4 is a sequence homology comparison showing that SE-WAP41 (SEQ ID NO:24), AtRGP1 (SEQ ID NO:19), AtRGP2 (SEQ ID NO:20), AtRGP3 (SEQ ID NO:21), AtRGP4 (SEQ ID NO:22) and PsRGP1 (SEQ ID NO: 23) share high amino acid sequence identity. Alignment was made with CLUSTAL X (Thompson et al., 1997). Black boxes denote identical amino acids, gray boxes denote similar amino acids.

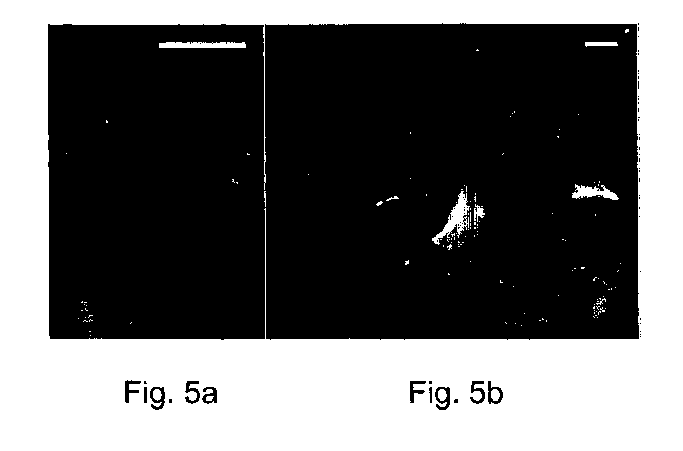

FIGs. 5a-b illustrate localization of transiently expressed AtRGP2:GFP in tobacco epidermal cells 48 hpi by Agrobacterium. Figure 5a - punctate fluorescence can be seen along cell walls as Paired fluorescence foci spanning common wall of adjacent cells. Figure 5b - a projection of several sections showing AtRGP2:GFP both in fluorescent bodies within cell cytoplasm, representing Golgi vesicles and in paired fluorescence foci spanning cell walls. Bars = 10µm.

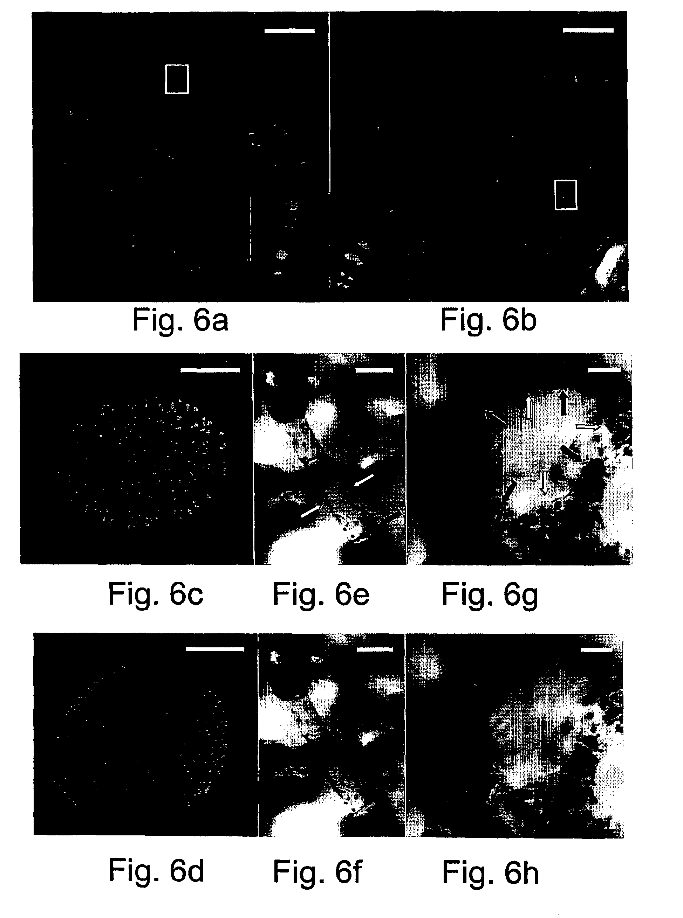

FIGs. 6a-h illustrate stable expression of AtRGP2:GFP and of MPTMV:GFP in transgenic tobacco plants. Identical fluorescence patterns are presented by leaf epidermal cells in AtRGP2:GFP (Figure 6a) and MPTMV:GFP (Figure 6b) expressing transgenic plants: punctate fluorescence spans walls as elongated bars, or paired foci, or appears as single foci inside the cell wall. The areas inside the white boxes are enlarged in the inserts in (Figure 6a) and (Figure 6b). The trichome-epidermis interface in AtRGP2:GFP (Figure 6d) and MPTMV:GFP (Figure 6c) expressing plants displays identical fluorescence patterns. In trichome (Figure 6f) and spongy mesophyll (SM) cells (Figure 6h) of AtRGP2:GFP expressing transgenic tobacco, fluorescence is detected only in wall areas where there is cell-cell contact (black arrows) and is absent from wall areas without cell-cell contact (white arrows). To emphasize wall partitions, the same trichome (Figure 6e) and spongy mesophyll cells (Figure 6g) are shown with the fluorescence channel turned off: black arrows indicate wall areas where there is cell-cell contact while white arrows indicate wall areas without cell-cell contact. SM denotes spongy-mesophyll cells while IS denotes inter-cellular spaces. Bars = 20µm.

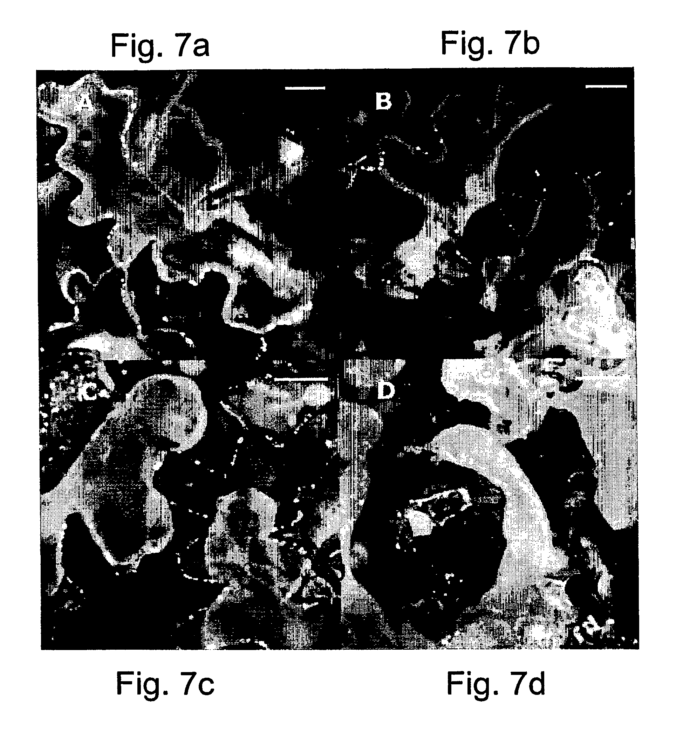

FIGs. 7a-d illustrate that both AtRGP2:GFP and MPTMV:GFP but not GONST1:YFP remain inside cell walls in plasmolyzed cells. The cytoplasmic marker DsRed1 (shown in magenta) was transiently expressed in leaf epidermal cells ∼72 h prior to plasmolysis. The DsRed1 labeled cytoplasm is appressed to cell walls prior to plasmolysis in AtRGP2:GFP expressing transgenic plants (Figure 7a) and retracts from walls of plasmolyzed cells in both AtRGP2:GFP expressing transgenic plants (Figure 7b) and MP^{TMV}:GFP expressing transgenic plants (Figure 7c). Both GFP fusion proteins (shown in green) remains inside cell walls in areas where the protoplast retracts from junction-walls in both AtRGP2:GFP (Figure 7b) and MP^{TMV}:GFP (Figure 7c) transgenic plants. In contrast, in GONST1:YFP expressing transgenic plants, GONST1:YFP (shown in yellow) does not remain inside cell walls in areas where the protoplast retracts from junction-walls (Figure 7d). Bars = 20µm.

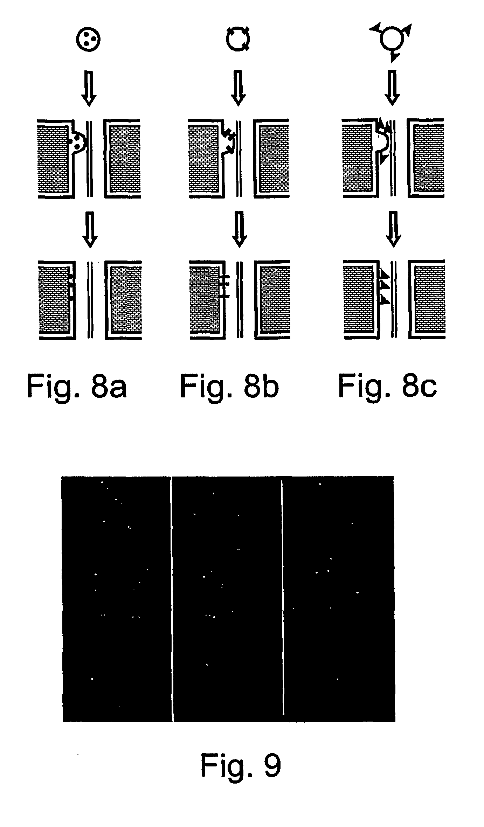

FIGs. 8a-c illustrates how a protein's association with the Golgi determines plasmadesmatal destination. Figure 8a - proteins residing in the Golgi lumen are exposed to the cell wall surrounding the plasmodesmata. Figure 8b - proteins with trans-membrane regions arrive at the plasma membrane delineating the plasmodesmata. Figure 8c - proteins peripherally associated with the cytosolic side of the Golgi membrane reach the inner side of the plasma membrane facing the cytoplasmic sleeve of the plasmodesmata.
FIG. 9 is a series of captured video frames showing Golgi-vesicles like movement of transiently expressed AtRGP2:GFP in tobacco epidermal cells;

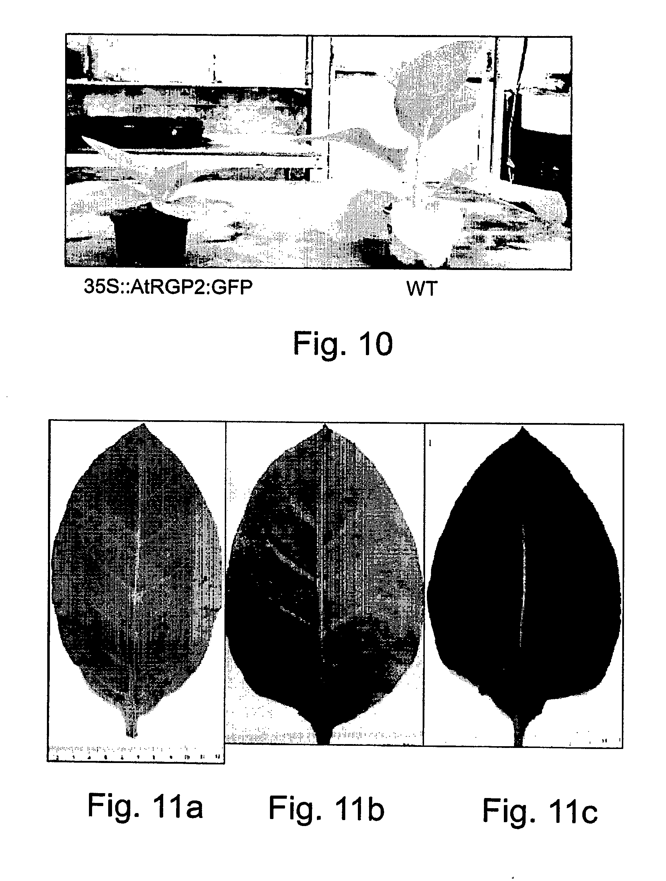

FIG. 10 illustrate morphological difference between WT and transgenic 35S::AtRGP:GFP overexpressing *N*. *tabacum* (NN) plants. Here WT are a week younger than transgenic plants, but both have the same number of leaves - that is they are of same physiological age. The internodes of transgenic plants are shorter than those of WT.
FIGs. 11 a-c illustrate necrotic lesions on leaves of *N. tabacum* (NN) 4 days after inoculation with TMV. Figure 11a illustrates a leaf of leaf pair three of WT plant showing the numerous regular, round shaped lesions. Figure 11b illustrates a leaf of leaf pair three of transgenic line 1-6 expressing 35S::AtRGP2:GFP; this leaf has less lesions than on WT and the lesions are irregular in shape and smaller in size. Figure 11c illustrates a mock treated leaf which shows no necrotic lesions or any damage at all.

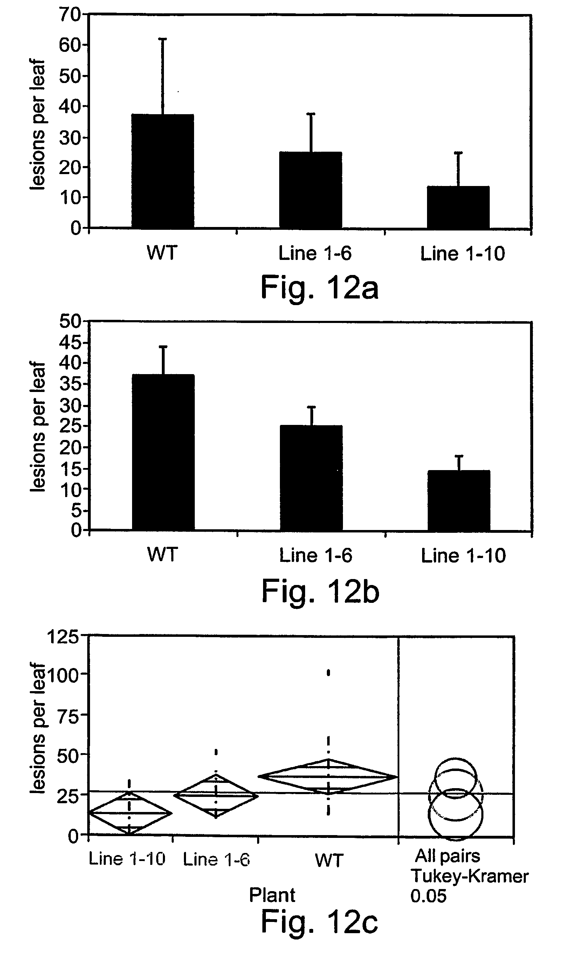

FIGs. 12a-c depict number of necrotic lesions on leaf pair three of TMV-inoculated WT *N. tabacum* (NN) and transgenic NN plants expressing 35S::AtRGP2:GFP. The number of lesions on each inoculated leaf (2 leaves on each plant) was counted and averaged, and the values for 13 WT and 8 transgenic plants were averaged and presented with SD (Figure 12a). Although there is a big variability in lesions number, the number of lesions on WT plants were significantly higher than on transgenic line 1-10 (Figure 12b). Means of standard error are shown in Figure 12c (P<0.05). The number of lesions on leaf pair two of WT plants was still higher than on leaf pair three (data not shown).

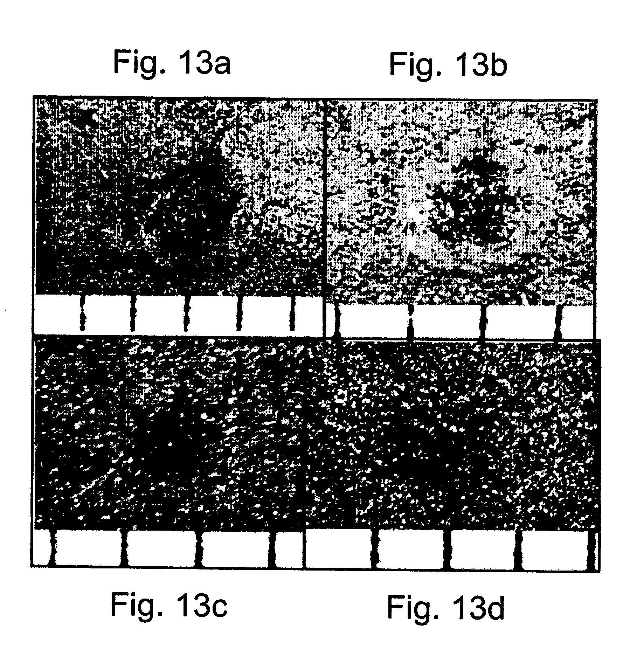

FIGs. 13a-d illustrate dry necrotic lesions on leaves of TMV-inoculated N. *tabacum* (NN) plants. Lesion on leaf pair two (Figure 13a) and three (Figure 13b) of WT plant are of the same size and have round and clear margins; three characteristic regions of the lesion could be distinguished. In contrast, the lesions on leaf pair three of 35S::AtRGP:GFP expressing transgenic line 1-6 (Figure 13c) and line 1-10 (Figure 13d) appear to be slightly amorphic in shape and smaller in size as compared to the lesions of the WT plants shown in Figures 13a-b. The lesions of the transgenic plants have no clear margins and are undistinguishable from each other. All lesions are photographed under the stereoscopic microscope 21 days following inoculation, from leaves preserved in 4°C. The dashed stripe under each picture is a millimetric scale. All lesions shown are from adaxial surface of the leaf and they are the same in shape and size also for abaxial surface (not shown).

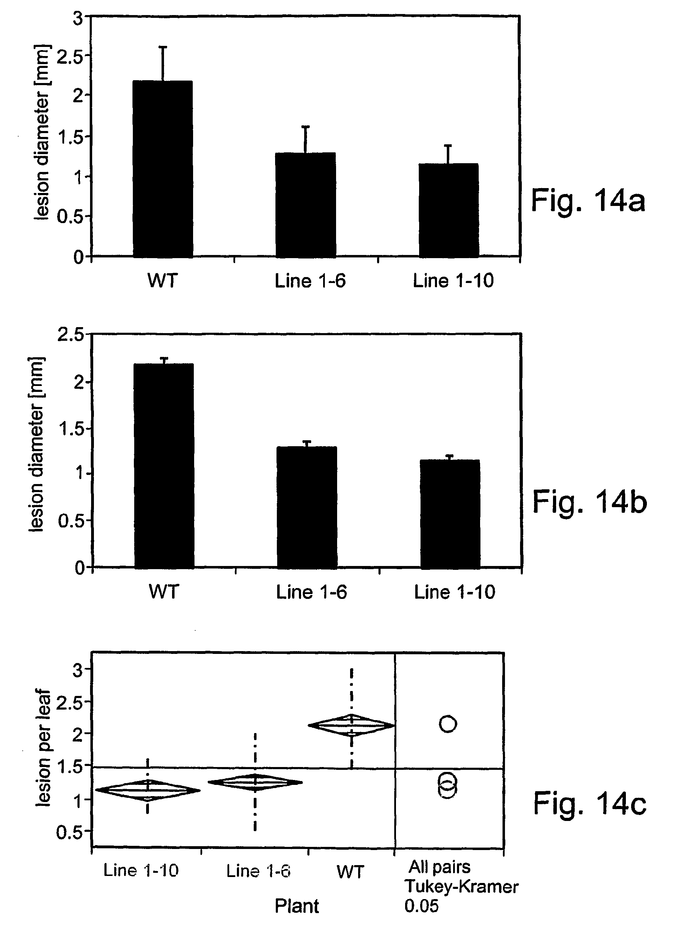

FIGs. 14a-c illustrate necrotic lesion diameter of TMV inoculated WT and transgenic (35S::AtRGP:GFP overexpressing) *N. tabacum* (NN) plants. The diameter of necrotic lesions from 3-4 inoculated leaf pair three of WT and two transgenic lines was measured and the average values with SD are presented. Mean lesion diameter of both transgenic lines (1-6 and 1-10) are smaller than of WT plants (Figure 14a). This difference in lesion diameter is statistically significant between WT and both transgenic lines (Figure 14b). The same data but standard error is shown in Figure 14c (P<0.05).

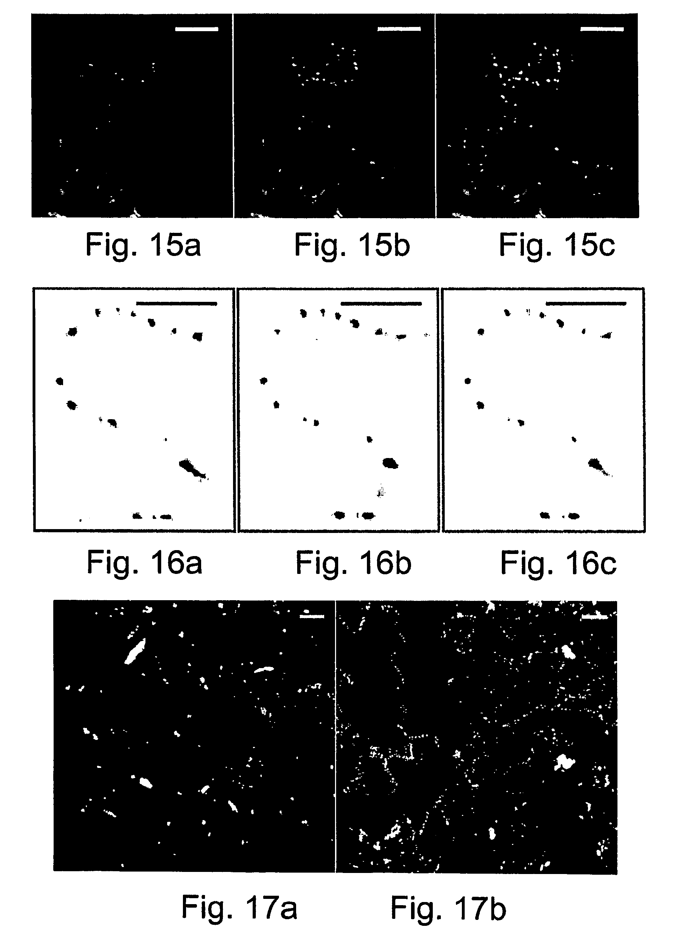

FIGs. 15a-c illustrate co-localization of AtRGP2:CFP and the Golgi apparatus marker GONST1:YFP. Co-localization is shown in a projection of 5 optical sections through a tobacco leaf epidermal cell transiently co-expressing AtRGP2:CFP and GONST1:YFP. AtRGP2:CFP fluorescence is shown in Figure 15a, GONST1:YFP fluorescence is shown in Figure 15b and both are shown (overlaid) in Figure 15c. Bars = 20µm. Optical sections were taken through the upper cortical cytoplasm near the cuticle where there are no Pd. Fluorescence seen in stomatal guard cells at the right edge of Figure 15 is autofluorescence.
FIGs. 16a-c illustrate co-localization of AtRGP2:GFP and aniline blue stained callose around Pd of transgenic tobacco. AtRGP2:GFP fluorescence is shown in Figure 16a aniline blue stained callose fluorescence is shown in Figure 16b both are overlaid in Figure 16c. Images show a section of cell wall between epidermal cells. Bars = 20µm.
FIGs. 17a-b illustrate the effect of Brefeldin A on AtRGP2:GFP labeling of both Golgi and Pd. Projections of 10 optical sections through the abaxial side of tobacco leaf epidermal cells treated with BFA (Figure 17a) or mock treated (Figure 17b). Bars = 20µm

### DESCRIPTION OF THE PREFERRED EMBODIMENTS

Disclosed are plants exhibiting altered plasmodesmatal conductance and of methods and constructs of generating same.

The principles and operation of the present invention may be better understood with reference to the drawings and accompanying descriptions.

Plasmodesmata interconnect neighboring plant cells and facilitate direct cytoplasmic cell-to-cell transport of molecules through the cell walls.

A direct relation between viruses and plasmodesmata was first demonstrated by electron microscopy during the late 1960s by Esau and co-workers, since then, numerous studies have demonstrated that plant viruses utilize plasmodemata as a conduit for cell to cell spread.

While investigating plasmodesmatal biochemistry, the present inventors have uncovered a 41kDa protein (Epel et al., 1996) which is associated with the membranous portion of the plasmodesmata. As is shown by the Examples set forth herein, the present inventors have demonstrated that this protein belongs to the Reversibly Glycosylated Polypeptides (RGPs) protein family, and that members of this family of proteins are capable of altering plasmodesmatal conductance.

Thus, according to one aspect of the present invention there is provided a method of altering plasmodesmatal conductance in plant tissue.

As is used herein, the phrase "altering plasmodesmatal conductance" refers to increasing or decreasing the ability of plasmodesmata to transport molecules or viruses therethrough. As is further described hereinunder the present inventors envisage several approaches for altering plasmodesmatal conductance.

The method is effected by regulating an expression level of a reversibly glycosylated polypeptide (RGP) or a functional portion thereof in cells of the plant tissue.

As used herein, the phrase "plant tissue" refers to isolated plant tissue (e.g. leaves, roots, flowers, stems or portions thereof etc) or to plant tissue which forms a part of a whole plant. Examples of plants which are suitable for use with the present invention include, but are not limited to, tomato, potato, cucumber, rice, maize, wheat, oats, barley, rye, soybean, canola, rape and cotton.

Regulation of an expression level of a reversibly glycosylated polypeptide can be utilized to decrease plasmodesmatal conductance.

Generation of plant tissue or whole plants which exhibit decreased plasmodesmatal conductance can be effected by a method of upregulating an expression level of an RGP in the plant cell.

Such upregulation can be achieved by upregulating an expression of an endogenous RGP or by expressing an exogenous RGP or a functional portion thereof in plant cells.

Upregulation of expression of an endogenous RGP can be effected via gene knock-in approaches. By introducing a strong promoter upstream of the endogenous RGP gene, one can induce over-expression of the coded RGP. Gene Knock-in techniques in plant cells are reviewed by Tzfira and White (2005).

In-plant expression of an exogenous RGP or a functional portion thereof is presently preferred. The RGP expressed can be for example, AtRGP1 (AF013627; SEQ ID NO:13), AtRGP2 (AF013628; SEQ ID NO:8), AtRGP3 (AF034255; SEQ ID NO:25), AtRGP4 (AF329280; SEQ ID NO:26), PsRGP1 (U31565; SEQ ID NO:27) and SE-WAP41 (U89897; SEQ ID NO:28), additional RGPs are listed in Table 1 below. The functional portion of an RGP is included within a C-terminal portion of an RGP, for example, amino acids 270-end (C-terminal amino acid) of SEQ ID NOs: 19-24.

Preferably, the expressed RGP (or a functional portion thereof) is linked to a bulking polypeptide (polypeptide sequence which add bulk, examples given below). By expressing in plant a polynucleotide sequence which include a sequence encoding at least a functional portion of RGP translationally fused to a sequence encoding a bulking polypeptide sequence such as Green fluorescent protein one can effectively decrease plasmodesmatal conductance. A bulking polypeptide will cause a decrease in the size of the cytoplasmic sleeve and thus create a nanobarrier which would be proportional to the size of the fusion protein. Very large RGP fusion proteins (e.g. RGP fused to Gus, td-tomato, td-tomato:GUS) can be used to progressively decrease the conductivity of the cytoplasmic sleeve (which is approximately 10 nm in diameter) to allow transport of only small molecules such as simple carbohydrates. Expression of an exogenous RGP or a portion thereof can also be used to decrease plasmodesmatal conductance to a point which supports selective conductance, e.g., allowing transport of molecules up to a known size or Stokes radius.

As is illustrated in the Examples section which follows, the present inventors have shown for the first time, that plant cells expressing a bulking polypeptide (e.g. GFP) targeted to the plasmodesmata are characterized by decreased plasmodesmata conductance. Thus, plasmodesmatal targeting of a bulking polypeptide via any plasmodesmata targeting moiety can be used to effectively decrease plasmodesmatal conductance.

Downregulation of an expression level of a reversibly glycosylated polypeptide (RGP) endogenous to the plant tissue can be utilized to increase plasmodesmatal conductance. Any plant endogenous reversibly glycosylated polypeptide can be targeted by the present invention, preferred are class 1 RGPs listed in Table 1 below.

**Table 1**

| **GenBank Accession No.** | **GI** | **Protein Description** | **Species** |
|---|---|---|---|
| AF013627, AAC50000, AAP68280 | 2317728, 2317729, 31711848 | AtRGP1 [reversibly glycosylated polypeptide-1] | *Arabidops*is *thaliana* |
| AF013628, AAC50001 | 2317730, 2317731 | AtRGP2 [reversibly glycosylated polypeptide-2] | *Arabidopsis thaliana* |
| AF034255, NP_187502, AAF07834 | 11863237, 30680679, 6403494 | AtRGP3 [reversibly glycosylated polypeptide-3 (RGP3)] | *Arabidopsis thaliana* |
| AF329280, AA050727 | 14326033, 28827766 | AtRGP4 [putative UDP-glucose] | *Arabidopsis thaliana* |
| BAD93611 | 62149103 | [hypothetical protein] | *Cucumis melo* |
| CAC83750 | 18077708 | [reversibly glycosylated polypeptide] | *Gossypium hirsutum* |
| AAT08665 | 47026881 | [reversibly glycosylated polypeptide] | *Hyacinthus orientalis* |
| AAT44738 | 48478827 | [UDP-glucose:protein transglucosylase-like protein SIUPTG1] | *Lycopersicon esculentum* |
| CAA77235 | 4158221 | [reversibly glycosylated polypeptide] | *Oryza sativa (indica cultivar-group)* |
| XP_479089 | 50939123 | [putative reversibly glycosylated polypeptide] | *Oryza sativa (japonica cultivar-group)* |
| CAA09469, NP_919052 | 3646373, 34915190 | [RGP1 protein] | *Oryza sativa (japonica cultivar-group)* |
| AAR13306 | 38194918 | [reversibly glycosylated protein] | *Phaseolus vulgaris* |
| O04300 | 34582497 | [Alpha-1,4-glucan-protein synthase [UDP-forming] (UDP-glucose:protein transglucosylase) (UPTG) (Reversibly glycosylated polypeptide)] | *Pisum sativum* |
| CAH59419 | 53748445 | [hypothetical protein] | *Plantago major* |
| Q8RU27 | 34582499 | [Alpha-1,4-glucan-protein synthase [UDP- forming] 2 (UDP-glucose:protein transglucosylase 2) (UPTG 2)] | *Solamum tuberosum* |
| Q9SC19 | 34582500 | [Alpha-1,4-glucan-protein synthase [UDP-forming] 1 (UDP-glucose:protein transglucosylase 1) (UPTG 1)] | *Solanum tuberosum* |
| CAA77237 | 4158232 | [reversibly glycosylated polypeptide] | *Triticum aestivum* |
| T11576 | 7488888 | [type IIIa membrane protein cp-wap11 - cowpea] | *Vigna unguiculata* |
| T11577 | 7488889 | [type IIIa membrane protein cp-wap 13 -cowpea] | *Vigna unguiculata* |
| P80607 | 34588146 | [Alpha-1,4-glucan-protein synthase [UDP-forming] (UDP-glucose:protein transglucosylase) (UPTG) (Amylogenin) (Golgi associated protein se-wap41)] | *Zea mays* |

Downregulation of an expression level of an endogenous RGP can be effected on the genomic and/or the transcript level using a variety of molecules that interfere with transcription and/or translation (e.g., antisense, siRNA, Ribozyme, or DNAzyme), or on the protein level using, e.g., antibodies, enzymes that cleave the polypeptide, and the like.

Following is a non-comprehensive list of agents/approaches suitable for downregulating expression level and/or activity of a plant endogenous RGP.

One example of an agent capable of downregulating an endogenous RGP is an antibody or antibody fragment capable of specifically binding an epitope of the RGP.
As used herein, the term "epitope" refers to any antigenic determinant on an antigen to which the paratope of an antibody binds.

Epitopic determinants usually consist of chemically active surface groupings of molecules such as amino acids or carbohydrate side chains and usually have specific three-dimensional structural characteristics, as well as specific charge characteristics.

Methods of producing polyclonal and monoclonal antibodies as well as fragments thereof in plants are well known in the art (see, for example, Harlow and Lane, 1988).

Antibody fragments according to the present invention can be prepared by in-plant expression of DNA encoding the fragment.

Fᵥ fragments comprise an association of variable heavy (VH) and variable light (VL) chains. This association may be noncovalent, as described in Inbar et al. (1972). Alternatively, the variable chains can be linked by an intermolecular disulfide bond or cross-linked by chemicals such as glutaraldehyde. Preferably, the Fv fragments comprise VH and VL chains connected by a peptide linker. These single-chain antigen binding proteins (sFv) are prepared by constructing a structural gene comprising DNA sequences encoding the VH and VL domains connected by an oligonucleotide. The structural gene is inserted into an expression vector, which is subsequently introduced into the plant cell (further description of plant expressible constructs is provided hereinbelow).

Another form of antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick and Fry (1991).

Another agent capable of downregulating a plant endogenous RGP is a small interfering RNA (siRNA) molecule used in the process of RNA interference (RNAi). RNAi is a two-step process, in the first, the initiation step, input double-stranded (dsRNA) is digested into 21- to 23-nucleotide (nt) small interfering RNAs (siRNAs), probably by the action of Dicer, a member of the RNase III family of dsRNA-specific ribonucleases, which processes (cleaves) dsRNA (introduced directly or by means of a transgene or a virus) in an ATP-dependent manner. Successive cleavage events degrade the RNA to 19- to 21-bp duplexes (the siRNA), each with 2-nucleotide 3' overhangs (Hutvagner and Zamore, 2002; and Bernstein, 2001).

In the second step, termed the effector step, the siRNA duplexes bind to a nuclease complex to form the RNA-induced silencing complex (RISC). An ATP-dependent unwinding of the siRNA duplex is required for activation of the RISC. The active RISC then targets the homologous transcript by base-pairing interactions and cleaves the RNA into 12-nucleotide fragments from the 3' terminus of the siRNA (Hutvagner and Zamore, 2002; Hammond et al., 2001; Sharp, 2001). Although the mechanism of cleavage remains to be elucidated, research indicates that each RISC contains a single siRNA and an RNase (Hutvagner and Zamore, 2002).

Because of the remarkable potency of RNAi, an amplification step within the RNAi pathway has been suggested. Amplification could occur by copying of the input dsRNAs to generate more siRNAs, or by replication of the siRNAs formed. Alternatively or additionally, amplification could be effected by multiple turnover events of the RISC (Hammond et al., 2001, Sharp, 2001; and Hutvagner and Zamore, 2002). For more information on RNAi, see the following reviews: Tuschl (2001); Cullen, (2002); and Brantl, S. (2002).

Synthesis of RNAi molecules suitable for use with the present invention can be effected as follows. First, the RGP mRNA sequence is scanned downstream of the AUG start codon for AA-dinucleotide sequences. Occurrence of each AA and the 19 3'-adjacent nucleotides is recorded as a potential siRNA target site. Preferably, siRNA target sites are selected from the open reading frame (ORF), as untranslated regions (UTRs) are richer in regulatory protein binding sites. UTR-binding proteins and/or translation initiation complexes may interfere with binding of the siRNA endonuclease complex (Tuschl, 2001). It will be appreciated, however, that siRNAs directed at untranslated regions may also be effective, as demonstrated for GAPDH, wherein siRNA directed at the 5' UTR mediated about a 90% decrease in cellular GAPDH mRNA and completely abolished protein levels (www.ambion.com/techlib/tn/91/912.html).

Second, potential target sites are compared to an appropriate genomic database (e.g., Arabidopsis, human, mouse, rat, etc.) using any sequence alignment software, such as the BlastN software available from the NCBI server (www.ncbi.nlm.nih.gov/BLAST/). Putative target sites that exhibit significant homology to other coding sequences are filtered out.

Qualifying target sequences are selected as templates for siRNA synthesis.
Preferred sequences are those including low G/C content, as these have proven to be more effective in mediating gene silencing as compared with sequences including G/C content higher than 55%. Several target sites are preferably selected along the length of the target gene for evaluation. For better evaluation of the selected siRNAs, a negative control is preferably used in conjunction. Negative-control siRNAs preferably include the same nucleotide composition as the siRNAs but lack significant homology to the genome. Thus, a scrambled nucleotide sequence of the siRNA is preferably used, provided it does not display any significant homology to any other gene.

Suitable RGP-directed siRNA are exemplified by SEQ ID NOs: 32-33.

Another agent capable of downregulating a plant endogenous RGP is a DNAzyme molecule, which is capable of specifically cleaving an mRNA transcript or a DNA sequence of the RGP. DNAzymes are single-stranded polynucleotides that are capable of cleaving both single- and double-stranded target sequences (Breaker and Joyce, 1995; Santoro and Joyce, 1997). A general model (the "10-23" model) for the DNAzyme has been proposed. "10-23" DNAzymes have a catalytic domain of 15 deoxyribonucleotides, flanked by two substrate-recognition domains of seven to nine deoxyribonucleotides each. This type of DNAzyme can effectively cleave its substrate RNA at purine:pyrimidine junctions (Santoro and Joyce, 1997); for review of DNAzymes, see: Khachigian, (2002).

Examples of construction and amplification of synthetic, engineered DNAzymes recognizing single- and double-stranded target cleavage sites are disclosed in U.S. Pat. No. 6,326,174 to Joyce et al. DNAzymes of similar design directed against the human Urokinase receptor were recently observed to inhibit Urokinase receptor expression, and successfully inhibit colon cancer cell metastasis in vivo (Itoh, T. et al., Abstract 409, American Society of Gene Therapy 5th Annual Meeting (www.asgt.org), June 5-9, 2002, Boston, Mass. USA.). In another application, DNAzymes complementary to bcr-ab1 oncogenes were successful in inhibiting the oncogene's expression in leukemia cells, and in reducing relapse rates in autologous bone marrow transplants in cases of Chronic Myelogenous Leukemia (CML) and Acute Lymphoblastic Leukemia (ALL).

Downregulation of a plant endogenous RGP can also be effected by using an antisense polynucleotide capable of specifically hybridizing with an mRNA transcript encoding the RGP.

Design of antisense molecules that can be used to efficiently downregulate an RGP must be effected while considering two aspects important to the antisense approach. The first aspect is delivery of the oligonucleotide into the cytoplasm of the appropriate cells, while the second aspect is design of an oligonucleotide that specifically binds the designated mRNA within plant cells in a manner inhibiting the translation thereof.

Algorithms are available for identifying those sequences with the highest predicted binding affinity for their target mRNA based on a thermodynamic cycle that accounts for the energetics of structural alterations in both the target mRNA and the oligonucleotide (see, for example, Walton et al., 1999).

Such algorithms have been successfully used to implement an antisense approach in cells. For example, the algorithm developed by Walton et al. enabled scientists to successfully design antisense oligonucleotides for rabbit beta-globin (RBG) and mouse tumor necrosis factor-alpha (TNF-alpha) transcripts. The same research group has more recently reported that the antisense activity of rationally selected oligonucleotides against three model target mRNAs (human lactate dehydrogenase A and B and rat gp130) in cell culture as evaluated by a kinetic PCR technique proved effective in almost all cases, including tests against three different targets in two cell types with phosphodiester and phosphorothioate oligonucleotide chemistries.

In addition, several approaches for designing and predicting efficiencies of specific oligonucleotides using an in vitro system were also published (Matveeva et al., 1998).

A suitable antisense oligonucleotide targeted against the RGP mRNA is exemplified by SEQ ID NOs:34-35.

Several studies have demonstrated the feasibility, and activity of antisense oligonucleotides in plant cells [see, for example, Tang et al., 2005; Smigocki, A.C., Wilson, D. (2004) and Sheehy et al.,1988.

Thus, the current consensus is that recent developments in the field of antisense technology, which, as described above, have led to the generation of highly accurate antisense design algorithms and a wide variety of oligonucleotide delivery systems, enable an ordinarily skilled artisan to design and implement antisense approaches suitable for downregulating expression of known sequences without having to resort to undue trial and error experimentation.

Another agent capable of downregulating a plant endogenous RGP is a ribozyme molecule capable of specifically cleaving an mRNA transcript encoding an RGP. Ribozymes increasingly are being used for the sequence-specific inhibition of gene expression by the cleavage of mRNAs encoding proteins of interest (Welch, P. J. et al. (1998). The possibility of designing ribozymes to cleave any specific target RNA has rendered them valuable tools in both basic research and commercial applications (Lutzelberger and Kjems 2006).

It will be appreciated that a non-functional analogue of at least a catalytic or Pd-association portion of an RGP can be also used as an agent which downregulates the expression levels of an endogenous RGP, via a dominant negative effect.

Each of the upregulating or downregulating agents described hereinabove can be expressed from a nucleic acid construct which is introduced into the plant cell using one of several known approaches. It will be appreciated however, that some of these agents (e.g. antibodies) can also be directly introduced into the plant cells via, for example, injection.

The nucleic acid construct utilized to express the upregulating or downregulating agents of the present invention can be any plant-capable expression construct.

Examples of suitable expression vectors include, but are not limited to, pCAMBIA vectors (available from CAMBIA) and Gateway vectors (available from Invitrogen).

The plant promoter employed by the constructs of the present invention can be a constitutive promoter, a tissue specific promoter, an inducible promoter or a chimeric promoter.

Examples of constitutive plant promoters include, without being limited to, CaMV35S and CaMV19S promoters, FMV34S promoter, sugarcane bacilliform badnavirus promoter, CsVMV promoter, *Arabidopsis* ACT2/ACT8 actin promoter, *Arabidopsis* ubiquitin UBQ1 promoter, barley leaf thionin BTH6 promoter, and rice actin promoter.

Examples of tissue specific promoters include, without being limited to, bean phaseolin storage protein promoter, DLEC promoter, PHS promoter, zein storage protein promoter, conglutin gamma promoter from soybean, AT2S1 gene promoter, ACT11 actin promoter from *Arabidopsis,* napA promoter from *Brassica napus* and potato patatin gene promoter.

The inducible promoter is a promoter induced by a specific stimuli such as pathogenic stress, include, without being limited to, the hsr203J and str246C active in pathogenic stress.

Preferably the promoter utilized by the present invention is a strong constitutive promoter such that over expression of the construct inserts is effected following plant transformation.

There are various methods of introducing nucleic acid constructs into both monocotyledonous and dicotyledenous plants (Potrykus, I. 1991; Shimamoto *et al.,* 1989). Such methods rely on either stable integration of the nucleic acid construct or a portion thereof into the genome of the plant, or on transient expression of the nucleic acid construct in which case these sequences are not inherited by a progeny of the plant.

In addition, several methods exist in which a nucleic acid construct can be directly introduced into the DNA of a DNA containing organelle such as a chloroplast.

There are two principle methods of effecting stable genomic integration of exogenous sequences such as those included within the nucleic acid constructs of the present invention into plant genomes:
(i) *Agrobacterium*-mediated gene transfer: (Klee *et al.* 1987 Klee and Rogers (1989); Gatenby,AA . (1989).
(ii) direct DNA uptake: Paszkowski *et al.,* 1989; including methods for direct uptake of DNA into protoplasts, Toriyama, K. *et al.* (1988). DNA uptake induced by brief electric shock of plant cells: Zhang *et al.* 1988;. Fromm *et al.* (1986). DNA injection into plant cells or tissues by particle bombardment, Klein *et al.* 1988; McCabe *et al.* 1988; Sanford. 1990; by the use of micropipette systems: Neuhaus et *al.,* 1987); Neuhaus and Spangenberg, 1990; or by the direct incubation of DNA with germinating pollen, DeWet *et al.* 1985; and Ohta, 1986.

The *Agrobacterium* system includes the use of plasmid vectors that contain defined DNA segments that integrate into the plant genomic DNA. Methods of inoculation of the plant tissue vary depending upon the plant species and the *Agrobacterium* delivery system. A widely used approach is the leaf disc procedure which can be performed with any tissue explant that provides a good source for initiation of whole plant differentiation. Horsch *et al.* 1988. A supplementary approach employs the *Agrobacterium* delivery system in combination with vacuum infiltration. The *Agrobacterium* system is especially viable in the creation of transgenic dicotyledenous plants.

There are various methods of direct DNA transfer into plant cells. In electroporation, protoplasts are briefly exposed to a strong electric field. In microinjection, the DNA is mechanically injected directly into the cells using very small micropipettes. In microparticle bombardment, the DNA is adsorbed on microprojectiles such as magnesium sulfate crystals, tungsten particles or gold particles, and the microprojectiles are physically accelerated into cells or plant tissues.

Following transformation plant propagation is exercised. The most common method of plant propagation is by seed. Regeneration by seed propagation, however, has the deficiency that due to heterozygosity there is a lack of uniformity in the crop, since seeds are produced by plants according to the genetic variances governed by Mendelian rules. Basically, each seed is genetically different and each will grow with its own specific traits. Therefore, it is preferred that the transformed plant be produced such that the regenerated plant has the identical traits and characteristics of the parent transgenic plant. Therefore, it is preferred that the transformed plant be regenerated by micropropagation which provides a rapid, consistent reproduction of the transformed plants.

Transient expression methods which can be utilized for transiently expressing the isolated nucleic acid included within the nucleic acid construct of the present invention include, but are not limited to, microinjection and bombardment as described above but under conditions which favor transient expression, and viral mediated expression wherein a packaged or unpackaged recombinant virus vector including the nucleic acid construct is utilized to infect plant tissues or cells such that a propagating recombinant virus established therein expresses the non-viral nucleic acid sequence.

Viruses that have been shown to be useful for the transformation of plant hosts include CaMV, TMV and BV. Transformation of plants using plant viruses is described in U.S. Pat. No. 4,855,237 (BGV), EP-A 67,553 (TMV), Japanese Published Application No. 63-14693 (TMV), EPA 194,809 (BV), EPA 278,667 (BV); and Gluzman, Y. et al., Communications in Molecular Biology: Viral Vectors, Cold Spring Harbor Laboratory, New York, pp. 172-189 (1988). Pseudovirus particles for use in expressing foreign DNA in many hosts, including plants, is described in WO 87/06261.

Construction of plant RNA viruses for the introduction and expression of non-viral exogenous nucleic acid sequences in plants is demonstrated by the above references as well as by Dawson, *et al.,* 1989; Takamatsu 1987; French 1986; and Takamatsu 1990.

When the virus is a DNA virus, the constructions can be made to the virus itself. Alternatively, the virus can first be cloned into a bacterial plasmid for ease of constructing the desired viral vector with the foreign DNA. The virus can then be excised from the plasmid. If the virus is a DNA virus, a bacterial origin of replication can be attached to the viral DNA, which is then replicated by the bacteria. Transcription and translation of this DNA will produce the coat protein which will encapsidate the viral DNA. If the virus is an RNA virus, the virus is generally cloned as a cDNA and inserted into a plasmid. The plasmid is then used to make all of the constructions. The RNA virus is then produced by transcribing the viral sequence of the plasmid and translation of the viral genes to produce the coat protein(s) which encapsidate the viral RNA.

Construction of plant RNA viruses for the introduction and expression in plants of non-viral exogenous nucleic acid sequences such as those included in the construct of the present invention is demonstrated by the above references as well as in U.S. Pat. No. 5,316,931.

In one embodiment, a plant viral nucleic acid is provided in which the native coat protein coding sequence has been deleted from a viral nucleic acid, a non-native plant viral coat protein coding sequence and a non-native promoter, preferably the subgenomic promoter of the non-native coat protein coding sequence, capable of expression in the plant host, packaging of the recombinant plant viral nucleic acid, and ensuring a systemic infection of the host by the recombinant plant viral nucleic acid, has been inserted. Alternatively, the coat protein gene may be inactivated by insertion of the non-native nucleic acid sequence within it, such that a protein is produced. The recombinant plant viral nucleic acid may contain one or more additional non-native subgenomic promoters. Each non-native subgenomic promoter is capable of transcribing or expressing adjacent genes or nucleic acid sequences in the plant host and incapable of recombination with each other and with native subgenomic promoters. Non-native (foreign) nucleic acid sequences may be inserted adjacent the native plant viral subgenomic promoter or the native and a non-native plant viral subgenomic promoters if more than one nucleic acid sequence is included. The non-native nucleic acid sequences are transcribed or expressed in the host plant under control of the subgenomic promoter to produce the desired products.

In a second embodiment, a recombinant plant viral nucleic acid is provided as in the first embodiment except that the native coat protein coding sequence is placed adjacent one of the non-native coat protein subgenomic promoters instead of a non-native coat protein coding sequence.

In a third embodiment, a recombinant plant viral nucleic acid is provided in which the native coat protein gene is adjacent its subgenomic promoter and one or more non-native subgenomic promoters have been inserted into the viral nucleic acid. The inserted non-native subgenomic promoters are capable of transcribing or expressing adjacent genes in a plant host and are incapable of recombination with each other and with native subgenomic promoters. Non-native nucleic acid sequences may be inserted adjacent the non-native subgenomic plant viral promoters such that said sequences are transcribed or expressed in the host plant under control of the subgenomic promoters to produce the desired product.

In a fourth embodiment, a recombinant plant viral nucleic acid is provided as in the third embodiment except that the native coat protein coding sequence is replaced by a non-native coat protein coding sequence.

The viral vectors are encapsidated by the coat proteins encoded by the recombinant plant viral nucleic acid to produce a recombinant plant virus. The recombinant plant viral nucleic acid or recombinant plant virus is used to infect appropriate host plants. The recombinant plant viral nucleic acid is capable of replication in the host, systemic spread in the host, and transcription or expression of foreign gene(s) (isolated nucleic acid) in the host to produce the desired protein.

Thus, the present invention provides methods which can be utilized to generate plants exhibiting altered plasmodesmatal conductance.

Alteration of plasmodesmatal conductance of plant cells can be utilized for the following:
(i) Pathogen resistance - by decreasing plasmodesmatal conductance, one can restrict or isolate viral spread to infected tissues thus reducing viral pathogenicity and the overall effect of the virus on plant viability.
(ii) Viral expression of commercially important polypeptides - plants expressing an RGP or a portion thereof, or a RGP-fusion protein can be utilized for viral expression of a commercially important molecule (e.g. insulin, Endostatin, lymphopoietin, aprotinin). Plasmodesmatal conductance is limited by the RGP, the RGP fusion protein or a portion thereof in a manner which enables Pd transport of the commercially important product expressed from the recombinant virus to harvested plant tissues (e.g. fruit), while trapping the virus particles at the site of infection and thus preventing transport thereof to the harvested portion of the plant.
(iii) Altering organ sizes (Fruits/flowers/tubers etc) - by tissue-specific expression of RGP fusion proteins that would alter the function of plasmodesmata in specific tissues and thus influence carbohydrate partitioning [see Olesinski et al., 1996.

As used herein the term "about" refers to ± 10 %.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following

### examples.

### EXAMPLES

Reference is now made to the following examples, which together with the above descriptions, illustrate the invention in a non limiting fashion.

### EXAMPLE 1

### Class 1 Reversibly Glycosylated Polypeptides ((C1)RGPs) modify plasmodesmatal conductance

### METHODS

### Plant Material

Zea mays L. cv. Jublee (Roger Bros. Co., Idaho Falls, USA) seeds were imbibed overnight and grown in moist vermiculite in the dark, for 5 days at 25°C. Nicotiana tabacum cv. Samson and cv. Xanthi plants were grown in 10 cm pots on a mix of equal volumes of soil and vermiculite (Pecka Hipper Gan, Rehovot, Israel) at 25°C under long day conditions (16-h-light/8-h-dark cycles).

### Extraction and Micro-Sequencing of SE-WAP41

The cell wall fraction (CW) was prepared from the first upper cm of mesocotyl of etiolated Z. mays seedlings as previously described (Epel et al., 1996). CW from 40gr of fresh tissue were incubated overnight at 4°C in 50ml of SE-buffer (3M NaCl, 5.6% 17 -mercaptoethanol, 50mM Hepes pH 7.4) and the salt-extracted (SE) proteins were separated from the CW by centrifuging at 12,000RPM for five minutes.

The pelleted walls were than washed with equal volume (50ml) of extraction buffer without the NaCl, the walls were removed by centrifugation as above and the supernatants were combined. Following desalting and concentration by ultrafiltration with a 100kDa transfer cut off (Amicon, Millipore, Billerica, USA), the proteins were separated by SDS-PAGE as previously described (Epel et al., 1996). The 41kDa band, containing approximately 11µg protein, was cut from the gel. Following trypsin digestion in-gel, the peptides were extracted from the gel, separated by RF-HPLC and micro-sequenced by automated Edman degradation by Eurosequence, Netherlands.

### Isolation of SE WAP41 from an Expression Library

A cDNA library constructed in the ZAP II (Stratagene, La Jolla, USA) with poly (A) RNA from meristematic tissues of mesocotyl and first leaf of 5-day-old etiolated maize seedlings was screened with purified S-41 polyclonal antiserum at a dilution of 1:2000 obtained as previously described (Epel et al., 1996). The S-41 antiserum employed was first purified on a Protein A column, and then on a Sepharose 4B column linked to total bacterial proteins (Harlow and Lane, 1988). An alkaline phosphatase linked goat-anti-rabbit antibody (Jackson Immuno Research Laboratories Inc., West Grove, PA), diluted 1:4000, was used to identify plaques expressing proteins recognized by S-41 by a color reaction in the presence of BCIP and NBT (Sambrook et al., 1989). All DNA procedures were performed by standard techniques (Sambrook et al., 1989). Phage isolates were converted to plasmid clones following the Stratagene ZAP excision protocol. Hybridisation groups were determined on nitrocellulose filters containing plaques of all isolated clones by hybridization at high stringency conditions with cDNA of an arbitrarily selected clone radioactively 18 labeled by a Random Primed DNA labeling kit (Boehringer Mannheim, Mannheim, Germany). The screening of clones by PCR was performed on isolated plaques using a degenerated forward primer 5'-TTYTTYCANCCWTAYCAYCT-3' (SEQ ID NO:1) where Y=A or G, W=A or T and N= A or G or C or T, and reverse primer complementary to sequence of flanking vector region (reverse T7 primer 5'-TAATACGACTCACTATAGGG-3' SEQ ID NO:2).

### Preparation of Antiserum Against Recombinant SE-WAP41

For the expression of recombinant SE-WAP41, the cDNA was PCR amplified employing the forward primer 5'-CGAATTCATATGGCGGGCACGGTGACG-3' (SEQ ID NO:3) and the reverse primer 5'-CCGGATCCTACTTTGGCCTTGCCATTTGC-3' (SEQ ID NO:4) which contained flanking NdeI and BamHI sites respectively and the PCR product was cloned into corresponding sites of pET16 (Novagen, Darmstadt, Germany) in fusion to the polyHis (His₁₀ SEQ ID NO:5). The resulting plasmid pET16:SE-WAP41 was transformed to BL21(DE3) cells and expression of His₁₀:SE-WAP41 was induced by 2mM IPTG in log phase cultures for four hours. The fusion protein was affinity-purified on Ni- NTA-Agarose beads (Qiagen, Hilden, Germany), released with imidazole and then rechromatographed, and the bound protein cleaved in column from the His tag by Factor Xa (Sigma). The purified protein was mixed with complete Freund's adjuvant (Difco Laboratories, Detroit MI, U.S.A) and the mixture was injected into 10-20 sites on the backs of one-month old female New Zealand rabbits. The subsequent four booster injections, spaced at four-weeks interval, were prepared with incomplete Freund's adjuvant.

### Preparation of Soluble and Membranal Cellular Fractions

Cell extracts were prepared and fractionated as previously described (Yahalom et al., 1991) with modifications: the filtrates from first homogenization and after nitrogen disruption bomb were combined and centrifuged for 1 hour at 90,000xg at 4°. The 19 supernatant was collected for analysis of soluble proteins. The pellet was resuspended in buffer HM/7.5 (20mM Tris-HCl pH 7.5, 0.25M sucrose, 10mM EGTA, 2mM EDTA and 1mM phenyl methyl sulphonyl fluoride [PMSF]), repelleted under the same conditions and used to extract membranal proteins.

### RNA transcript slot blot analysis

Plant material from three areas of etiolated seedlings mesocotyl was harvested, frozen in liquid nitrogen and total RNA was extracted using TRIzol reagent (GibcoBRL Life Technologies, Gaithersburg, USA) as described by manufacturer. Ten µg RNA were glycoxylated, separated in 1.4% agarose gel and passively transferred to a Hybond-N nylon filter (Amersham). Hybridisation was performed with random primed 32P-labelled probes at 65°C for SE-WAP41 containing the whole cDNA including 3' and 5' noncoding sequence and for maize 26S-rRNA and at 55°C for actin from soybean in a solution containing 0.26M phosphate buffer pH 7.2, 7% SDS, 1 mM EDTA, 1% BSA. Filters were washed at 61 °C, first in 0.26M phosphate buffer pH 7.2, containing 1% SDS, then in 2xSSC, 0.1% SDS, and finally in 1XSSC, 0.1%SDS. The 26S-rRNA gene was used as a loading control. Scanning was done with a Fuji BAS 1000 phosphoimager (Fuji, Japan).

### Construction of Plant Expression Plasmids

To create plasmids pBinGFP, pBinDsRed1 and pBinCFP, cassettes containing the Cauliflower mosaic virus 35S promoter, omega sequence enhancer (SEQ ID NO:6.), a synthetic GFP (sGFP) gene (SEQ ID NO:29) (Chiu et al., 1996) or the DsRed1 gene (Clontech Laboratories Inc.) (SEQ ID NO:30) or the ECFP gene (Clontech Laboratories Inc.) (SEQ ID NO:31) respectively and the nitric oxide synthase transcriptional terminator (SEQ ID NO:7), were cloned into the HindIII-EcoRI sites of pBINPLUS (Vanengelen et al., 1995). To construct plasmids pBinAtRGP2:GFP and pBinAtRGP2:CFP, the AtRGP2 gene (SEQ ID NO:8), was amplified without it's stop codon, by PCR performed with a 5'-TCTAGAATTCATGGTTGAGCCGGCGAATACTG-3' (SEQ ID NO:9) 20 forward primer containing XbaI and EcoRI sites and a 5'-TCTAGATACTGCTCTCAAGCTTTTGCCACTG-3' (SEQ ID NO:10) reverse primer containing an XbaI site and cloned into a unique XbaI site present in pBinGFP or pBinCFP respectively between the omega enhancer sequence and the sGFP/ECFP start codon. EcoRI digestion was used to verify AtRGP2 orientation in pBinAtRGP2:GFP and pBinAtRGP2:CFP. Plasmid pBinAtRGP3:GFP was constructed in the same way using a 5'-TCTAGAATTCATGGCGCAATTGTATTCTTCCGTG-3' (SEQ ID NO: 11) forward primer and a 5'- TCTAGAATTTTTGCCTTTTGGTGCCTCAGC-3' (SEQ ID NO: 12) reverse primer. Construction of pBinAtRGP1:GFP was identical, except AtRGP1 (SEQ ID NO: 13), was amplified with a 5'-CCTAGGAATTCATGGTTGAGCCGGCGAACAC-3' (SEQ ID NO:14) forward primer containing AvrII and EcoRI sites and a 5'-CCTAGGAGCTTTAGTGGGTGGGTTAAGCTC-3' (SEQ ID NO:15) reverse primer containing an AvrII site and AvrII-AtRGP1-AvrII fragment was cloned into the unique XbaI site in pBinGFP. Plasmid pBinAtRGP4:GFP was constructed in the same way as pBinAtRGP1:GFP using a 5'-CCTAGGAATTCATGGCGGGCTACAACCTCGAAGCTATC-3' (SEQ ID NO:16) forward primer and a 5'-CCTAGGCTTGGCCTTGACATCTTTGCCATGGCTC-3' (SEQ ID NO: 17) reverse primer.

### Transient Expression

Transient expression was induced using Agrobacterium leaf injection (Lavy et al., 2002). The lower face of N. tabacum cv. Samson or cv. Xanthi leaves was injected with Agrobacterium tumefacienes strain GV3101 harboring the indicated plasmids, using a 1ml syringe without a needle. Observation of transient expression was limited to epidermal cells at the lower face of the leaves. GFP fluorescence was examined 24 to 48 h after injection. DsRed1 fluorescence was examined ∼72 h after injection.

### Stable Transformations of Tobacco

To create AtRGP2:GFP or GONST1:YFP expressing transgenic plants, wild-type N. tabacum cv. Samson were transformed by Agrobacterium tumefacienes strain GV3101 harboring pBinAtRGP2:GFP or pBinGONST1:YFP plasmids accordingly, using a modified leaf disk method (Gallois and Marinho, 1995). Leaf disks were incubated in Agrobacterium for 30 minutes, washed and placed for co-cultivation in Murashugy Skoog medium containing 2µg/ml kinetin and 0.8µg/ml IAA. The rooting medium, contrary to Gallois and Marinho, did not contain hormones. Analysis was performed on leaves of T0 plants.

### Callose Staining and Co-localization with AtRGP2:GFP

Callose was stained by incubating leaf segments for 40 minutes in a mixture of 0.1% aniline blue in DDW and 1M glycine PH = 9.5 at a volume ratio of 2:3 respectively and premixed at least one day before use. Co-localization was viewed with a DMRBE fluorescence light microscope (Leica); aniline blue-stained callose fluorescence was viewed with a BP 340 - 380 nm excitation filter, a RKP 400 dichromatic mirror and a LP 425 nm emission filter; AtRGP2:GFP fluorescence was viewed with a BP 450 - 490 nm excitation filter, a RKP 510 dichromatic mirror and a BP 515 - 560 nm emission filter. GFP and aniline blue monochrome photographs were converted to magenta and yellow respectively using Zeiss LSM 5 image browser and color images were inverted using Adobe Photoshop 7.0 ME to obtain green and blue images respectively on white background.

### Plasmolysis

Leaves stably expressing either AtRGP2:GFP or MPTMV:GFP were plasmolyzed by incubation in 30% glycerol. Leaf sections were mounted on microscope slides in the plasmolysis solution. DsRed 1 was transiently expressed in the leaves by Agrobacterium leaf injection ∼72 h before they were plasmolyzed.

### Treatment with Brefeldin A

During transient expression of AtRGP2:GFP, at ∼22 hours post Agrobacterium injection, the abaxial side of tobacco leaves was injected with 50 µg/ml BFA in 0.1% DMSO on one side of the major vein while the opposite side of the major vein was mock injected with 0.1% DMSO. Injections were performed using 1ml syringes without a needle, so that the intercellular spaces beneath the epidermis were completely filled. Leaf segments located at equal distances to either side of the major vein and at the same area along the baso-petal axis were cut and examined by confocal microscopy. Optical sections 0.9 µm thick through leaf epidermal cells were scanned by CLSM 2 to 5 hours post injection of BFA/DMSO (24 to 27 hours post Agrobacterium injection). Photographed cells did not include large cells at the base of trichomes nor small cells adjacent to stomatal guard cells. The experiment was repeated twice, with a total of 4 leaves from 4 plants. Fluorescently labeled Pd were counted in 10 BFA treated cells and 10 mock treated cells. In each cell the total number of fluorescently labeled Pd was counted in five consecutive optical sections representing the approximate middle layer of the cell.

### Fluorescence Confocal Microscopy

Fluorescent confocal microscopy was carried out using a Zeiss R510 confocal laser scanning microscope. GFP excitation was perforated with an argon laser set to 488nm and emission was detected with a 525nm±20nm BP-filter combination. CFP excitation was performed with an argon laser set to 458nm and emission was detected with a 500nm±25nm BP-filter. When YFP was used with CFP, YFP excitation was performed with an argon laser set to 514nm and emission was detected with a 550nm±20nm BP-filter combination. When YFP was used with DsRed1, YFP excitation was performed with an argon laser set to 488nm and emission was detected with a 525nm±20nm BP-filter combination. The different YFP conditions were used to prevent bleed-through between fluorescent channels. DsRed1 excitation was performed with a Helium-Neon laser set to 543nm, and emission was detected with a 587.5nm±27.5nm BP filter. In order to expose spongy mesophyll for imaging, leaves were torn by hand so that the epidermis at the lower face of leaf sections was peeled off. Image analysis was performed with Zeiss LSM-5 image browser.

### RESULTS

### SE-WAP41 is all RGP Protein

The present inventors have previously shown that S-41 immunolabeled Pd and Golgi (Epel et al., 1996). Since this antiserum was generated against a protein band from a SDS-PAGE gel, it may recognize more than one protein that could be present in the band. Furthermore, this antiserum cross-reacted with two additional proteins. A combination of experimental methods was used in order to molecularly characterize the protein or proteins recognized by S-41, which targets to Pd. In one approach, S-41 was utilized to screen an expression library generated using mRNA obtained from meristematic tissues of 5 days old maize seedlings. Screening approximately 4.3x105 plaque forming units yielded 40 clones recognized by S-41. Hybridization experiments revealed that the isolated clones represent two hybridization groups termed H1 and H2, containing 19 and 21 clones respectively, indicating that S-41 identified two distinct proteins from the expression library. In parallel, purified SE-WAP41, a salt extractable 41kDa Wall Associated Protein, that was recognized by S-41 (Epel et al., 1996) was micro-sequenced yielding a 25 amino acids long sequence: NLDFLGMWRPFFQPYHLIIVQDGDP (SEQ ID NO:18). Based on this sequence, a degenerative primer was synthesized to allow identification of isolated clones encoding the SE-WAP41 internal sequence. The degenerative primer was used in combination with a reverse primer from the library's vector sequence in a PCR reaction to identify clones previously isolated employing the S-41 antiserum and which encode a protein that contains the sequenced peptide. Seven clones were identified, all belonging to the H1 hybridization group, establishing that this hybridization group encodes the SE-WAP41 protein. The insert lengths of the various clones belonging to H1 were determined by an additional PCR reaction with primers complementary to vector sequences on both sides of the insert and the clone that had the longest insert was sequenced. The open reading frame of the insert encodes a protein containing 364 amino acids with a calculated mass of 41,038 Daltons, matching the size of the 41 kDa wall-associated protein. The predicted gene product contained the internal sequence previously obtained by SE-WAP41 micro-sequencing, conforming that the clone encoded SE- WAP41. The sequence of the maize gene encoding SE-WAP41 protein was deposited in EMBL and NCBI data libraries (accession U89897). High sequence homology of 6 SE-WAP41 to members of the RGP protein family (pfam 03214) resulted in SE- WAP41 annotation as an RGP protein. A representative of the H2 hybridization group was sequenced and found to encode an aldolase protein (data not shown). Antiserum Raised Against Recombinant SE-WAP41 Uniquely Identifies a 41kDa Protein that is Highly Enriched in the Wall Fraction. SE-WAP41 expressed in E. coli as a his-tag fusion was used to immunize rabbits. The antiserum obtained from one rabbit, termed SR-41, exhibited good immunogenic activity to the purified recombinant protein in western blots. Western analysis with SR-41 of proteins from the soluble, the membranal and the wall fractions, demonstrated that SE-WAP41 is highly enriched in the wall fraction, compared to the membranal and soluble fractions (Figures 1a-c), The membrane and soluble fractions are more weakly labeled, suggesting lower levels of SE-WAP41 association with these fractions. This western analysis also verified that SR-41 antiserum recognizes the 41kDa protein (figure 1b), without recognizing the other major bands labeled by S-41 (Figure 1c).

### SE-WAP41 mRNA Expression Levels are Highest in Meristematic and Elongating Tissues

The formation of both simple primary and secondary Pd and their modification to branched Pd occurs primarily in meristematic tissues and in expanding and/or elongating tissues (Ehlers and Kollmann, 2001; Roberts and Oparka, 2003). If SE-WAP41 is a Pd associated protein, it is expected that in etiolated maize seedlings its expression should be highest in the mesocotyl node, which is a meristem tissue and in a one to two centimeter region just below the node, a region in which cells are undergoing extensive elongation. Thus, SE-WAP41 mRNA expression levels were compared in segments, each 1 cm in length, along the mesocotyl developmental gradient. Northern analysis using SE-WAP41 cDNA probe showed that SE-WAP41 transcript level was highest in the region containing the mesocotyl node and the most extensively expanding cells, and decreased along the mesocotyl developmental gradient (Figures 2a-c).

The correlation between cell division and mRNA expression level was further examined in a second experimental system. Exposure of etiolated seedlings to light brings about a cessation of cell divisions in the mesocotyl meristem (Iino, 1982; Yahalom et al., 1987). Northern analysis was used to examine SE-WAP41 mRNA expression levels in maize mesocotyls at different times following exposure to light of etiolated seedlings. Following exposure to light, SE-WAP41 mRNA levels decreased 2 fold after 3 hours, 3.5 fold after 6 hours and 10 fold after 24 hours (Figure 3b). Actin mRNA levels displayed a similar decrease (Figures 3a) indicative of cell division cessation.

### Transiently Expressed AtRGP: GFPs Associate with Pd and Golgi Vesicles.

A further study was done with genes of Arabidopsis, since its genome has been fully sequenced and open reading frames annotated. The Arabidopsis genome encodes four homologous genes termed AtRGP1, AtRGP2, AtRGP3 and AtRGP4 (SEQ ID NOs:19-21 respectively) which belong to class 1 reversibly glycosylated proteins (C1 RGPs). These Arabidopsis proteins show high degrees of amino acid sequence identity with SE-WAP41 (Figure 4). Amino acid sequence analysis (http://www.ncbi.nlm.nih.gov/BLAST/) revealed that AtRGP2 is the closest homologue of SE-WAP41 sharing 87% amino acid sequence identity. The AtRGP2 coding sequence was fused to a GFP coding sequence and the AtRGP2:GFP chimera was transiently expressed in tobacco leaf epidermal cells using Agrobacterium leaf injection. Confocal microscopy revealed fluorescence in several distinct sites inside epidermal cells. Punctate paired fluorescent foci were detected on opposite sides of the cell wall - a pattern consistent with AtRGP2 being a plasmodesmal-associated protein (Figures 5a-b). Fluorescence was also observed as small fluorescence foci throughout the cell cytoplasm (Figure 5b), which moved rapidly throughout the cytoplasm and along cytoplasmic strands through the vacuole, in a manner characteristic of Golgi vesicles (Figure 9). PsRGP1 a C1RGPs that shares high sequence identity with SE_WAP41 and AtRGP proteins (Figure 4) immunolocalizes to the trans Golgi apparatus (Dhugga et al., 1997): this supports the supposition that AtRGP2:GFP mobile foci represent Golgi bodies. In order to verify that the mobile foci are indeed Golgi bodies, Golgi Nucleotide Sugar Transporter 1 (GenBank Accession number NM_179621) Fused to Yellow Fluorescence protein (GONST1:YFP) which has been shown to label Golgi bodies (Baldwin et al., 2001) was employed. AtRGP2 when fused to Cyan Fluorescence Protein (AtRGP2:CFP) and co-expressed with GONST1:YFP clearly co-localize with GONST1:YFP (Figures 15a-c). Fluorescence could also be seen in large amorphous areas inside cells, apparently representing inclusion bodies resulting from the massive quantities of AtRGP2:GFP produced inside the cells during transient expression and in a small area inside cell nuclei (data not shown). In order to examine whether plasmodesmal association is a general characteristic of C1RGPs, AtRGP1:GFP, AtRGP3:GFP and AtRGP4:GFP were constructed and transiently expressed in tobacco leaf epidermal cells. These fusion proteins displayed fluorescence patterns identical to that presented by AtRGP2:GFP in all respects (data not shown), except that AtRGP1:GFP fluorescence levels were considerably lower. These fluorescence patterns are consistent with AtRGP1, AtRGP3 and AtRGP4 being plasmodesmal-associated proteins. The observation that all four Arabidopsis RGP proteins examined appear to be plasmodesmal-associated indicates that plasmodesmal association is a general characteristic of members of C1RGPs.

### Stably Expressed AtRGP2: GFP is Plasmodesmal-Associated

Transgenic tobacco plants constitutively expressing the AtRGP2:GFP fusion under the control of the 35S promoter of the Cauliflower mosaic virus were generated. In these plants, AtRGP2:GFP fluorescence was localized in punctate fluorescence foci inside cell walls (Figure 6a). This pattern is similar to that presented by transgenic tobacco plants constitutively expressing a GFP fusion of a known Pd marker, the movement protein of Tobacco Mosaic Virus (MPTMV:GFP) (Figure 6b). The cell wall at the base of a trichome is especially rich in Pd. Transgenic MPTMV:GFP expressing tobacco plants exhibit a characteristic dense punctate pattern where these Pd are fluorescently marked (Figure 6c). The same pattern is presented by the transgenic AtRGP2:GFP expressing plants (Figure 6d) supporting the conclusion that AtRGP2 localizes to Pd. AtRGP2:GFP fluorescence was seen only in wall areas that contain Pd, where two cells are in contact, whereas wall areas devoid of Pd were unlabeled, as demonstrated by trichome cells and spongy mesophyll cells. Tobacco trichomes are hair-like structures, protruding from the epidermis surface, consisting of a single file of several cells (Figure 6e). Therefore, tobacco trichome cells have both Pd containing walls, those walls separating two cells, and lateral walls that are only in contact with the air surrounding the trichome, which are devoid of Pd. Spongy mesophyll cells (Figure 6g), likewise have regions with no cell-cell contact, where walls face an inter-cellular space and are devoid of Pd, and regions with cell-cell contact, where walls contain Pd. Fluorescence was detected only within cell wall regions where there is cell-cell contact, in both trichome cells (Figure 6f), and spongy mesophyll cells (Figure 6h). This differentiating labeling of walls further indicates that AtRGP2 is associated with Pd, or with the wall sheath surrounding Pd. In order to verify that the punctate fluorescence foci inside cell walls indeed represent Pd, callose staining by aniline blue was employed. Callose has been widely used as a plasmodesmal marker (Baluska et al., 1999; Gorshkova et al., 2003 and Bayer et al., 2004). When leaves of AtRGP2:GFP expressing transgenic tobacco were stained by aniline blue, AtRGP2:GFP clearly co-localized with the aniline blue - stained callose (Figures 16a-c) present around Pd. Wild type tobacco, not expressing GFP, when stained by aniline blue and photographed with GFP conditions displayed no fluorescently labeled Pd (Data not shown). Likewise, transgenic AtRGP2:GFP expressing transgenic tobacco not stained by aniline blue but photographed with aniline blue conditions also displayed no fluorescently labeled Pd (Data not shown). These negative control experiments verify that co-localization is not the result of fluorescence bleed through between GFP and aniline blue channels. In mature tobacco tissue, AtRGP2:GFP fluorescence was not detected in mobile bodies possibly due to the lower steady-state levels present during stable expression.

### AtRGP2:GFP Remains Inside Cell Walls of Plasmolyzed Cells

Plasmolysis of cells in leaves causes shrinkage of the cell protoplast and retraction of the plasma membrane from the wall with Pd remaining embedded in the cell wall (Turner et al., 1994). Plasmolysis was induced in leaves of the transgenic AtRGP2:GFP expressing tobacco plants in order to learn about the association of AtRGP2 with Pd. The fluorescent dye DsRed1, a cytoplasmic marker, was used to allow better visualization of the receding cytoplasm and plasma membrane during plasmolysis. DsRed1 was transiently expressed in leaf epidermal cells of transgenic tobacco plants stably expressing AtRGP2:GFP, before they were plasmolyzed, thus marking the cytoplasm of these cells (Figure 7a). Following plasmolysis, AtRGP2:GFP fluorescence clearly remained inside cell walls, in regions where the cytoplasm became disassociated from both sides of the cell wall (Figure 7b). This result demonstrates that the punctate sites are not within the cortical cytoplasm nor PM associated, but are embedded in the cell wall. This is similar to the result obtained in plasmolyzed transgenic tobacco plants stably expressing MPTMV:GFP (Figure 7c). In order to examine if association with Pd occurs for any stably expressed Golgi localized GFP fusion protein, GONST1:YFP expressing transgenic tobacco plants were generated. When these plants were plasmolyzed no fluorescence remained in cell walls (Figure 7d).

### Brefeldin A Inhibits Labeling of Pd by AtRGP2: GFP.

The presence of AtRGPs GFP fusions in both Pd and the Golgi apparatus led the present inventors to hypothesize that ^{CI}RGPs are delivered to Pd via the Golgi apparatus. Brefeldin A (BFA), a disruptor of the Golgi apparatus (reviewed by Nebenfuhr et al., 2002) was used to examine this hypothesis. Epidermal cells of tobacco leaves treated with BFA during transient expression of AtRGP2:GFP were compared with cells not treated with BFA. BFA treated cells displayed a decrease in the numbers of fluorescently labeled Golgi bodies (Figures 17a-b). Fluorescently labeled foci, containing labeled plasmodesma or clusters of plasmodesma in pit fields which are too close together to resolve individually, were counted in five consecutive confocal sections representing the middle layer of each cell. On average BFA treated cells presented 24.4±2.95 fluorescently labeled Pd, whereas mock treated cells presented 77.3±10.85 fluorescently labeled Pd. Thus, BFA treated cells displayed a decrease of approximately 3 fold in the average number of fluorescently labeled Pd when compared to cells not treated with BFA. Paired-sample T-test showed results to be significantly different at P<0.001. The fact that the number of AtRGP2:GFP - labeled Pd was lower in the presence of the Golgi - disrupting BFA, combined with the observation that GFP fusions of AtRGPs label the Golgi apparatus, demonstrates that ^{C1}RGPs pass through the Golgi apparatus prior to their arrival at Pd.

### EXAMPLE 2

### Over-expression of the Plasmodesma/-Associated Protein AtRGP2 Fused to Green Fluorescent Protein Impairs Cell-to-Cell Spread of Tobacco mosaic virus in Transgenic Tobacco Plants

The present inventors have shown that Class 1 Reversibly Glycosylated Polypeptides ((C1)RGPs) of *Arabidopsis thaliana* are plasmodesmal-associated proteins which are delivered to plasmodesmata (Pd) via the Golgi apparatus (Sagi *et al.,* 2005). The inventors have also presented and analyzed various data that indicates that the protein is an outer Golgi peripherally-associated membrane which targets to the inner plasmodesmal plasma membrane (Sagi et al., 2005).

To test the hypothesis that Class 1 Reversibly Glycosylated Polypeptides block plasmodesmatal conduction, WT and transgenic *Nicotiana tabacum* (NN) plants over-expressing GFP tagged (C1)AtRGP2 of *Arabidopsis* (AtRGP2:GFP) were inoculated with *Tobacco mosaic virus* (TMV). In this strain of tobacco a TMV virus that can replicate and spread cell to cell will give raise to a hypersensitive reaction (HR), which can be used as quantitative and qualitative assay for virus infectivity and spread.

### Materials and methods

*N. tabacum* cv Xanthi (NN) plants, both WT and two independent transgenic lines (homozygous for 35S::AtRGP2:GFP), were grown and inoculated with *Tobacco mosaic virus* (TMV) under the same physical conditions: T∼23°C, short day external (greenhouse) light conditions, daily watered. Due to a certain developmental delay of Transgenic lines, WT and transgenic plants were inoculated at the same physiological age (4-5 pairs of leaves) (Figure 1). WT plants were inoculated at age 2 months and transgenic plants at age 2.5 months after germination. Homozygous transgenic plants exhibited a dwarfed morphology.

Inoculation was carried out with immuno-purified whole TMV particles (stock concentration of 1mg/ml). This stock solution was further diluted with 0.05M sodium phosphate buffer (pH=7) to final working concentration of 10ng/ml (inoculum).
Second, third and/or fourth pairs of mature, fully developed leaves were chosen for inoculation. No more than four leaves on each plant were inoculated with virus. Corresponding leaf pairs of WT and transgenic plates that were approximately of the same size were used. Inoculation procedure was as follows: carborundum (320 Grit) was powdered uniformly onto the entire adaxial leaf surface and then 150µl drop of TMV inoculum (10ng/ml) was placed along the midrib, rubbed over the leaf surface and after 30-60sec the leaf was briefly rinsed with tap-water. Control (mock) inoculation was made with the same volume of sodium phosphate buffer alone.
Four days after inoculation, leaves were harvested and stored in 4°C. Leaves were photographed and the number of necrotic lesions counted. The diameter of lesions on WT and transgenic lines was also measured and photographed under a stereoscopic microscope.

### RESULTS

WT (13) and transgenic plants (8 from each line) were inoculated with TMV. Four days post inoculation (dpi) necrotic lesions were clearly seen both on WT and transgenic plants. The number of lesions per leaf on WT were higher than on transgenic plants over-expressing AtRGP2:GFP (Figures 11a-c and 12a-c).

Although there is large variability in number of lesions between leaves of both WT and transgenic lines, still differences are clearly seen between the number of necrotic lesions (Figures 12a-c) and their size and shape (Figures 13a-d).

The difference between WT and transgenic line 1-10 was statistically significant (Figure 12b). The shape and size of the lesions differed between WT and transgenic plants (Figure 13a-d and 14a-c). The diameter of necrotic lesions on WT plants was higher than on two transgenic lines (Figure 14a), and this difference is statistically significant (Figure 14b).

The variability in lesions number could be due to uneven covering of the leaf surface by carborundum and/or unequal pressure on the leaf during inoculation. However, the shape and size of necrotic lesions are obviously different between WT and transgenic plants. WT plants have characteristically big and round shaped lesions with clear margins, and their three distinct regions could be recognized (Figure 13a-b). The lesions on both transgenic lines are smaller in size, have irregular shapes, not round as on WT and with unclear diffuse margins (Figure 13c-d). The lesions on the second pair of leaves of WT plants are larger in diameter and higher in number than on the third pair (Figure 14a-c). This is because the second leaf is more developed and is bigger than the third. Therefore, only the third pair of leafs, which approximately are of the same size for both WT and transgenic plants could be compared (Figure 12a-c and 14a-c).

### CONCLUSIONS

The present study discloses the cloning and sequencing of a gene encoding a protein belonging to the RGP protein family pfam 03214 [The Conserved Domain Architecture Retrieval Tool (CDART)]. The RGP pfam 03214 is a family of highly conserved plant-specific proteins that are present in both monocotyledones (Gupta et al., 2000; Langeveld et al., 2002) and dicotyledones (Dhugga et al., 1997; Delgado et al., 1998; Bocca et al., 1999; Zhao and Liu, 2002). Members of this family are reversibly self-glycosylated in the presence of UDP-glucose, UDP-xylose and UDP-galactose (Dhugga et al., 1991; Dhugga et al., 1997; Delgado et al., 1998); glycosylation occurs on an Arg residue (Singh et al., 1995). Hydrophobic cluster analysis suggested that RGPs may be glycotransferases (Saxena and Brown, 1999).

RGPs have been subdivided into two classes termed herein as C1RGP and C2RGP. Members of C2RGP have been suggested to be similar to processive - glycotransferases which contain two conserved domains, designated domains A and B (Langeveld et al., 2002). Based on structure analysis, members of C1RGPs, to which SE-WAP41 belongs, have been suggested to be non- processive -glycotransferases as they possess domain A but lack domain B characteristic of processive - glycotransferases (Saxena and Brown, 1999).

Recent data suggest that members of this family, specifically members of C1RGPs, may function as UDP-sugar carriers, leading to the hypothesis that they function in delivering UDP-sugars from the cytoplasm to a transporter in the Golgi membrane (Faik et al., 2000; Porchia et al., 2002) where transmembrane - glycotransferases 12 function in transporting the UDP-sugars into the Golgi lumen. However the possibility that C1RGPs may be transported by the Golgi to the Pd and become incorporated into the Pd was not suggested. The present study conclusively shows that C1RGPs are plasmodesmal-associated proteins. Cell fractionation experiments showed SE-WAP41 enrichment in the Pd-containing wall fraction. Confocal microscopy of tobacco epidermal cells stably expressing AtRGP2:GFP or transiently expressing AtRGP1:GFP, AtRGP2:GFP, AtRGP3:GFP and AtRGP4:GFP revealed a fluorescence pattern of paired fluorescence foci on opposite sides of the cell wall similar to the fluorescence pattern presented by tobacco cells expressing the known Pd marker MPTMV:GFP. Moreover, in transgenic AtRGP2:GFP expressing tobacco, confocal microscopy of trichomes and spongy mesophyll cells showed that fluorescence foci were only present inside Pd containing cell walls and were absent from walls devoid of Pd. Colocalization of aniline blue-stained callose present around Pd with AtRGP2:GFP fluorescence foci inside cell walls demonstrates that these AtRGP2:GFP foci represent Pd.

Further support is provided by plasmolysis experiments. During plasmolysis the plasma membrane recedes with the cytoplasm detaching from cell walls, yet the Pd remain embedded in the cell wall (Turner et al., 1994). The fact that AtRGP2:GFP fluorescence remained inside cell walls of plasmolyzed epidermal cells even in regions where the cytoplasm became disassociated from both sides of the cell wall indicates that these proteins are plasmodesmal- associated. The fact that GONST1:YFP, another Golgi-localized fluorescent fusion protein, does not associate with Pd when stably expressed in transgenic tobacco, as demonstrated by plasmolysis, indicates that association with Pd is specific to ^{C1}RGPs and does not occur by some default pathway shared by any Golgi-localized fluorescent fusion protein.

Since Pd synthesis occurs mainly in meristematic and growing tissues, it is expected that C1RGPs synthesis and association with Golgi would occur in these regions. Indeed Northern analysis showed such a correlation, with higher SE-WAP41 mRNA levels measured in regions undergoing cell division and elongation.

The fact that all Arabidopsis C1RGP proteins appear to be plasmodesmal-associated, suggests that association with Pd may be a general characteristic of C1 RGPs. Evidence suggest that the Golgi apparatus serves as the vehicle by which C1 RGPs are delivered to Pd since PsRGP1 was previously immunolocalized to the trans Golgi (Dhugga et al., 1997) and Arabidopsis cell suspension fractionation data indicated AtRGP1 is localized to the Golgi apparatus (Delgado et al., 1998). Transient expression of AtRGP2:GFP shows that this fluorescent fusion protein is associated with mobile bodies, Golgi vesicles which are streaming within the cytoplasm. These mobile bodies were shown to be Golgi vesicles by the colocalization of AtRGP2:CFP with the known Golgi marker GONST1:YFP . The fact that the Golgi disrupting drug BFA greatly reduces the number of AtRGP2:GFP foci seen throughout the cytosol during transient expression further supports the conclusion that these foci represent Golgi vesicles. Most importantly, labeling of Pd by transiently expressed AtRGP2:GFP was reduced by approximately threefold in the presence of the Golgi disruptor BFA, demonstrating that passage through the Golgi apparatus is required for AtGRP2:GFP targeting to Pd. Since it is known that Golgi vesicles participate in the formation of both primary and secondary Pd it is conceivable that The Golgi apparatus serves as a vehicle for C1RGPs delivery to Pd.

The marked difference between the levels of AtRGP2:GFP in the Golgi and in Pd are probably due to permanent accumulation of AtRGP2:GFP in Pd, while in the Golgi, the protein is in transit, being shuttled by the Golgi network from the site of synthesis to its final destination in Pd. During transient expression, the cells ectopically produce very large quantities of AtRGP2:GFP in a very short time, enabling it's detection as fluorescence associated with the Golgi apparatus. Western blots of different cell fractions detected C1RGPs not only in membranes and wall fractions but also in the soluble fraction. Immunogold electron microscopy, however, detect almost no cytosolic RGP other than that associated with Golgi (Epel et al., 1996; Dhugga et al., 1997).

Golgi associated proteins can either reside in the Golgi lumen, have a trans-membrane region crossing the Golgi membrane, or be peripherally attached to the cytosolic side of Golgi membranes. For proteins delivered to Pd via Golgi vesicles, each of these possibilities would result in their arrival to different destinations within a plasmodesma. When Golgi vesicles fuse with the plasma membrane, their lumen spills outwards to the cell wall, while their cytosolic side faces the cytosol. Therefore, a protein residing in the Golgi lumen would be exported to the cell wall surrounding the plasmodesma (Figure 8a), while a protein with a trans-membrane region would become an integral Pd plasma- membrane protein with Golgi-lumen facing domains exposed to the apoplast (Figure 8b) and a protein peripherally associated with the cytosolic side of the Golgi membrane would reach the inner-side of the plasma membrane facing the cytoplasmic sleeve of the plasmodesma (Figure 8c). Present bioinformatic (http://smart.embl- heidelberg.de/ and http://psort.nibb.ac.jp), and biochemical data indicate that pea, maize and Arabidopsis C1RGPs are peripherally associated with the outer Golgi membrane (Dhugga et al., 1991; Epel et al., 1996; Dhugga et al., 1997; Delgado et al., 1998). It is therefore most likely that upon fusion of the Golgi to the PM of a plasmodesma or in the vicinity of a plasmodesma, C1RGPs would be attached to the PM facing the cytoplasmic sleeve of the Pd.

Regardless of its function it is clear that RGPs and in particular C1RGPs are transported to the plasmodesmata. This is true for all members of this class of proteins since, as shown herein, all of the AtRGP proteins utilized herein correctly targeted to the Pd when expressed in tobacco. Therefore it can be conclude that both the signal responsible for C1RGPs targeting to Pd and the mechanism of their delivery to Pd are general and species-independent.

The function of C1RPGs in plants, and in particular in plasmodesmata, was examined using a viral spread model and phenotypic observations.

For hypersensitive response to occur, a virus must replicate and spread to a minimal number of cells after initial replication in an infected plant cell. The decreased number of necrotic lesions and the decreased size of the lesions in the transgenic plants of the present invention indicates that C1RPGs alter plasmodesmatal conductance thereby inhibiting initial spread of virus particles through the plasmodesmata.

This inhibition results in a lower observed necrotic lesions in transgenic plants as compared to their WT counterparts. In addition, the transgenic plants of the present invention are also phenotypically distinguishable from WT plants in both height and internode length.

Provided are transgenic plants which are capable of localizing viral infection as well as having a commercially valuable phenotype.

### REFERENCES

Baldwin, T.C., Handford, M.G., Yuseff, M.I., Orellana, A., and Dupree, P. (2001). Identification and characterization of GONST1, a Golgi-localized GDP-mannose transporter in Arabidopsis. Plant Cell 13, 2283-2295.
Baluska, F., Samaj, J., Napier, R., and Volkmann, D. (1999). Maize calreticulin localizes preferentially to plasmodesmata in root apex. Plant J 19, 481-488.
Bayer, E., Thomas, C.L., and Maule, A.J. (2004). Plasmodesmata in Arabidopsis thaliana suspension cells. Protoplasma 223, 93-102.
Beachy, R.N., and Heinlein, M. (2000). Role of P30 in replication and spread of TMV. Traffic 1, 540-544.
Bernstein, E. (2001). Role for a bidentate ribonuclease in the initiation step of RNA interference. Nature 409, 363-366.
Bocca, S.N., Kissen, R., Rojas-Beltran, J.A., Noel, F., Gebhardt, C., Moreno, S., du Jardin, P., and Tandecarz, J.S. (1999). Molecular cloning and characterization of the enzyme UDP-glucose: protein transglucosylase from potato. Plant Physiology and Biochemistry 37, 809-819.
Botha, C.E.J., Hartley, B.J., and Cross, R.H.M. (1993). The Ultrastructure and Computer-Enhanced Digital Image-Analysis of Plasmodesmata at the Kranz Mesophyll-Bundle Sheath Interface of Themeda-Triandra Var Imberbis (Retz) Camus,A. In Conventionally-Fixed Leaf Blades. Ann Bot-London 72, 255-261.
Brantl, S. (2002). Antisense-RNA regulation and RNA interference. Biochim Biophys Acta 1575, 15-25.
Breaker, R.R., and Joyce, G.F. (1995). A DNA enzyme with Mg2+-dependent RNA phosphoesterase activity. Curr Biol 2, 655-660.
Chiu, W.L., Niwa, Y., Zeng, W., Hirano, T., Kobayashi, H., and Sheen, J. (1996). Engineered GFP as a vital reporter in plants. Curr. Biol. 6, 325-330.
Citovsky, V., and Zambryski, P. (2000). Systemic transport of RNA in plants. Trends Plant Sci. 5, 52-54.
Cullen, B.R. (2002). RNA interference: antiviral defense and genetic tool. Nat Immunol 3, 597-599;
Delgado, I.J., Wang, Z.H., de Rocher, A., Keegstra, K., and Raikhel, N.V. (1998). Cloning and characterization of AtRGP1 - A reversibly autoglycosylated Arabidopsis protein implicated in cell wall biosynthesis. Plant Physiol. 116, 1339-1349.
DeWet, J.M.J., Bergquist, R.R., Harlan, J.R., Brink, D.E., Cohan, C.E., Newell, C.A. and DeWet, A.E. 1985. Exogenous DNA transfer in maize (Zea mays) using DNA-treated pollen. p. 197-209. In: Experimental Manipulation of Ovule Tissue. Chapman, G. P., Mantell, S. H., Daniels, W. (Eds.). Longman, London.
Dhugga, K.S., Tiwari, S.C., and Ray, P.M. (1997). A reversibly glycosylated polypeptide (RGP1) possibly involved in plant cell wall synthesis: Purification, gene cloning, and trans-Golgi localization. P Natl Acad Sci USA 94, 7679-7684.
Dhugga, K.S., Ulvskov, P., Gallagher, S.R., and Ray, P.M. (1991). Plant Polypeptides Reversibly Glycosylated by Udp-Glucose - Possible Components of Golgi Beta-Glucan Synthase in Pea Cells. J. Biol. Chem. 266, 21977- 21984.
Ding, B., Turgeon, R., and Parthasarathy, M.V. (1992). Substructure of Freeze-Substituted Plasmodesmata. Protoplasma 169, 28-41.
Ding, B., Itaya, A., and Woo, Y.M. (1999). Plasmodesmata and cell-to-cell communication in plants. Int Rev Cytol 190, 251-+. Ehlers, K., and Kollmann, R. (2001). Primary and secondary plasmodesmata: structure, origin, and functioning. Protoplasma 216, 1-30.
Dawson WO Lewandowski DJ, Hilf ME, Bubrick P, Raffo AJ, Shaw JJ Grantham GL andDesjardins PR. (1989) Virology 172:285-292.
Ehlers, K., and Kollmann, R. (2001). Primary and secondary plasmodesmata: structure, origin, and functioning. Protoplasma 216, 1-30.
Epel, B.L., vanLent, J.W.M., Cohen, L., Kotlizky, G., Katz, A., and Yahalom, A. (1996). A 41 kDa protein isolated from maize mesocotyl cell walls immunolocalizes to plasmodesmata. Protoplasma 191, 70-78.
Faik, A., Desveaux, D., and Maclachlan, G. (2000). Sugar-nucleotide-binding and autoglycosylating polypeptide(s) from nasturtium fruit: biochemical capacities and potential functions. Biochemical Journal 347, 857-864.
French, R., Janda, Mi and Ahlquist, P. 1986. Bacterial gene inserted in an engineered RNA virus: efficient expression in monocotyledonous plant cells. Science 231: 1294-1297.
Fromm, M.E., Taylor, L.P. and Walbot, W. 1986. Stable transformation of maize after gene transfer by electroporation. Nature 319: 791-793.
Gafni, Y., and Epel, B.L. (2002). The role of host and viral proteins in intra- and inter-cellular trafficking of geminiviruses. Physiol Mol Plant P 60, 231-241.
Gallois, P., and Marinho, P. (1995). Leaf Disk Transformation Using Agrobacterium tumefacienes-Expression of Heterologous Genes in Tobacco. In Plant Gene Transfer and Expression Protocols, H. Jones, ed (Totowa: Humana Press Inc.
Gatenby, AA. (1989) Regulation and expression of plant genes in microorganisms in Plant Biotechnology, eds. Kung, S. and Arntzen, C. J., Butterworth Publishers, Boston, Mass. (1989) p. 93-112.
Gorshkova, E.N., Erokhina, T.N., Stroganova, T.A., Yelina, N.E., Zamyatnin, A.A., Kalinina, N.O., Schiemann, J., Solovyev, A.G., and Morozov, S.Y. (2003). Immunodetection and fluorescent microscopy of transgenically expressed hordeivirus TGBp3 movement protein reveals its association with endoplasmic reticulum elements in close proximity to plasmodesmata. J. Gen. Virol. 84, 985-994.
Gupta, P., Raghuvanshi, S., and Tyagi, A.K. (2000). PCR-amplification and cloning of the coding region of a cDNA for a reversibly glycosylated polypeptide from rice with possible involvement in the biosynthesis of glucans. Journal of Plant Biochemistry and Biotechnology 9, 99-102.
Hammond, S.M., Caudy, A.A., and Hannon, G.J. (2001). Post-transcriptional gene silencing by double-stranded RNA. Nat. Rev. Gen. 2:110-119.
Harlow, E.D., and Lane, D. (1988). Antibodies, a Laboratory Manual. (Cold Spring Harbor Laboratory Press). Haywood, V., Kragler, F., and Lucas, W.J. (2002). Plasmodesmata: pathways for protein and ribonucleoprotein signaling. Plant Cell 14 Suppl, S303-325.
Haywood, V., Kragler, F., and Lucas, W.J. (2002). Plasmodesmata: pathways for protein and ribonucleoprotein signaling. Plant Cell 14 Suppl, S303-325.
Heinlein, M. (2002). Plasmodesmata: dynamic regulation and role in macromolecular cell-to-cell signaling. Curr Opin Plant Biol 5, 543.
Heinlein, M., and Epel, B.L. (2004). Macromolecular transport and signaling through plasmodesmata. In International Review of Cytology - a Survey of Cell Biology, Vol 235,93-164.
Hepler, P.K. (1982). Endoplasmic-Reticulum in the Formation of the Cell Plate and Plasmodesmata. Protoplasma 111, 121-133.
Horsch RB, Fry J, Hoffman N, Neidermeyer J, Rogers SG, Fraley RT (1988). Leaf disc transformation. In: Gelvin SB, Schilperoort RA, Verma DPS (eds.), Plant Molecular Biology Manual, pp. A5/1-9.Dordrecht: Kluwer Academic Publishers.
Hutvagner, G. and Zamore, P.D. (2002). RNAi: Nature abhors a double-strand. Curr Opin Gen Dev 72, 225-232.
lino, M. (1982). Inhibitory-Action of Red-Light on the Growth of the Maize Mesocotyl - Evaluation of the Auxin Hypothesis. Planta 156, 388-395.
Inbar, D., Hochman, J., and Givol, D. (1972). Localization of Antibody-Combining Sites within the Variable Portions of Heavy and Light Chains. Proc Natl Acad Sci USA 69, 2659-2662.
Jones, M.G.K. (1976). The origin and development of plasmodesmata. In Intercellular communication in plants: studies on plasmodesmata, B.E.S. Gunning and A.W. Robards, eds (Berlin, Heidelberg, New York: Springer- Verlag), pp. 81-105.
Khachigian, L.M. (2002). DNAzymes: cutting a path to a new class of therapeutics. Curr Opin Mol Ther 4, 119-121.
Klee, H, Horsch, R and Rogers, S (1987) Agrobacterium-mediated plant transformation and its further applications to plant Biology. Annu. Rev. Plant Physiol. 38:467-486.
Klee, H and Rogers, S in Cell Culture and Somatic Cell Genetics of Plants, Vol. 6, Molecular Biology of Plant Nuclear Genes, eds. Schell, J., and Vasil, L. K., Academic Publishers, San Diego, Calif. (1989) p. 2-25.
Klein, TM, Gradziel, T, Fromm, ME and Sanford, JC (1988). Factors Influencing Gene Delivery into Zea Mays Cells by High-Velocity Microprojectiles. Bio/Technology 6:559-563.
Langeveld, S.M.J., Vennik, M., Kottenhagen, M., van Wijk, R., Buijk, A., Kijne, J.W., and de Pater, S. (2002). Glucosylation activity and complex formation of two classes of reversibly glycosylated polypeptides. Plant Physiol. 129, 278-289.
Larrick, J.W., and Fry, K.E. (1991). PCR Amplification of Antibody Genes. METHODS: A Companion to Methods in Enzymology 2(2), 106-110.
Lavy, M., Bracha-Drori, K., Sternberg, H., and Yalovsky, S. (2002). A cell- specific, prenylation-independent mechanism regulates targeting of type II RACs. Plant Cell 14, 2431-2450.
Lutzelberger M, Kjems J. (2006). Strategies to identify potential therapeutic target sites in RNA. Handb Exp Pharmacol. 173:243-59.
Matveeva, O., Felden, B., Tsodikov, A., Johnston, J., Monia, B.P., Atkins, J.F., Gesteland, R.F., and Freier, S.M. (1998). Prediction of antisense oligonucleotide efficacy by in vitro methods. Nature Biotechnology 16, 1374-1375.
Nebenfuhr, A., Ritzenthaler, C., and Robinson, D.G. (2002). Brefeldin A: Deciphering an enigmatic inhibitor of secretion. Plant Physiol. 130, 1102-1108.
McCabe, DE, Swain, WF Martinell, BJ and Christou, P. (1988). Stable Transformation of Soybean (Glycine Max) by Particle Acceleration. Bio/Technology 6:923-926.
Ohta, Y. 1986. High efficiency genetic transformation of maize by a mixture of pollen and exogenous DNA. Proc. Natl. Acad. Sci. USA 83: 715-719.
Oparka, K.J., Roberts, A.G., Boevink, P., Santa Cruz, S., Roberts, L., Pradel, K.S., Imlau, A., Kotlizky, G., Sauer, N., and Epel, B. (1999). Simple, but not branched, plasmodesmata allow the nonspecific trafficking of proteins in developing tobacco leaves. Cell 97, 743-754.
Overall, R.L. (1999). Structure of Plasmodesmata. In Plasmodesmata, Structure, Function, Role in Cell Communication, A.J. vanBel and W.J.P. vanKestern, eds (Berlin, Heidelberg, New York: Spring-Verlag), pp. 129-148.
Neuhaus, G., Spangenberg, G., Mittelsten Scheid, O. and Schweiger, H.G. 1987. Transgenic rapeseed plants obtained by microinjected DNA into microspore-derived embryoids. Theor. Appl. Genet. 75: 30-36.
Neuhaus, G. and G. Spangenberg. 1990. Plant transformation by microinjection techniques. Physiologia Plantarum. 79: 213-217.
Olesinski, A., Almon, E., Navot, N., Perl, A., Galun, G., Lucas, W.J. and Wolf, S. (1996). Tissue-specific expression of a tobacco mosaic virus movement protein in transgenic potato plants alters plasmodesmal function and carbohydrate partitioning. Plant Physiol 111:541-550.
Paszkowski, J., Saul, M.W. and Potrykus, I. 1989. Plant gene vectors and genetic transformation: DNA-mediated direct gene transfer to plants. p. 52-68. In: Cell Culture and Somatic Cell Genetics of Plants. Vol. 6. Molecular Biology of Plant Nuclear Genes. Schell, J. and Vasil, I. K. (Eds.). Academic Press, San Diego.
Porchia, A.C., Sorensen, S.O., and Scheller, H.V. (2002). Arabinoxylan biosynthesis in wheat. Characterization of arabinosyltransferase activity in Golgi membranes. Plant Physiol. 130, 432-441.
Potykus, I. (1991). Gene Transfer to Plants: Assessment of Published Approaches and Results, Annu. Rev. Plant. Physiol., Plant. Mol. Biol. 42:205-225.
Robards, A.W., and Lucas, W.J. (1990). Plasmodesmata. Annu Rev Plant Phys 41, 369-419.
Roberts, A.G., and Oparka, K.J. (2003). Plasmodesmata and the control of symplastic transport. Plant Cell Environ. 26, 103-124.
Sambrook, J., Fritsch, E.F., and Maniatis, T. (1989). Molecular Cloning: a Laboratory Manual. (Cold Spring Harbor Laboratory Press).
Sanford, J.C. 1990. Biolistic plant transformation. Physiol. Plant. 79: 206-209.
Santoro, S.W., and Joyce, G.F. (1997). A general purpose RNA-cleaving DNA enzyme. Proc Natl Acad Sci USA 94, 4262-4266).
Saxena, I.M., and Brown, R.M. (1999). Are the reversibly glycosylated polypeptides implicated in plant cell wall biosynthesis non-processive beta- glycosyltransferases? Trends Plant Sci. 4, 6-7.
Sharp, P.A. (2001). RNA interference--2001. Genes Dev 15, 485-490.
Sheehy, R.E., Kramer, M.H., William R. (1988). Reduction of polygalacturonase activity in tomato fruit by antisense RNA. P Natl Acad Sci USA 85(23): 8805-8809.
Shimamoto, K, Terada, R, Izawa, T, and Fujimoto, H (1989). Fertile transgenic rice plants regenerated from transformed protoplasts. Nature 338:274-276
Singh, D.G., Lomako, J., Lomako, W.M., Whelan, W.J., Meyer, H.E., Serwe, M., and Metzger, J.W. (1995). Beta-Glucosylarginine - a New Glucose Protein Bond in a Self-Glucosylating Protein from Sweet Corn. Febs Letters 376, 61- 64.
Smigocki, A.C., Wilson, D. (2004). Pest and disease resistance enhanced by heterologous suppression of a Nicotiana plumbaginifolia cytochrome P450 gene CYP72A2. Biotechnol Lett.26(23):1809-14
Takamatsu N, Ishikawa M, Meshi T and Okada Y.(1987). Expression of bacterial chloramphenicol acetyltransferase gene in tobacco plants mediated by TMV-RNA. EMBO J. 6:307-311.
Takamatsu N, Watanabe Y, Yanagi H, Meshi T, Shiba T, Okada Y (1990). "Production of enkephalin in tobacco protoplasts using tobacco mosaic virus RNA vector", FEBS Letters 269: 73-76.
Tang, W., Kinken, K., Newton, R.J. (2005). Inducible antisense-mediated post-transcriptional gene silencing in transgenic pine cells using green fluorescent protein as a visual marker. Plant Cell Physiol. 46(8): 1255-1263
Thompson, J.D., Gibson, T.J., Plewniak, F., Jeanmougin, F., and Higgins, D.G. (1997). The CLUSTAL_X windows interface: flexible strategies for multiple sequence alignment aided by quality analysis tools. Nucleic Acids Research 25,4876-4882.
Toriyama K, Arimoto Y, Uchimiya H, Hinata K (1988) Transgenic rice plants after direct gene transfer into protoplasts. Bio/technology 6: 1072-1074**.**
Turner, A., Wells, B., and Roberts, K. (1994). Plasmodesmata of maize root tips: structure and composition. J Cell Sci 107 (Pt 12), 3351-3361. 28
Tuschl, T. (2001). RNA interference and small interfering RNAs. ChemBioChem 2, 239-245.
Tzfira, T and White, C. (2005). Towards targeted mutagenesis and gene replacement in plants. Trends Biotechnol.23(12):567-9.
Vanengelen, F.A., Molthoff, J.W., Conner, A.J., Nap, J.P., Pereira, A., and Stiekema, W.J. (1995). Pbinplus - an Improved Plant Transformation Vector Based on Pbin19. Transgenic Res 4, 288-290.
Walton, S.P., Stephanopoulos, G.N., Yarmush, M.L., and Roth, C.M. (1999). Prediction of antisense oligonucleotide binding affinity to a structured RNA target. Biotechnol Bioeng 65, 1-9.
Welch, P. J. Barber JR and Wong-Staal F. (1998). Expression of ribozymes in gene transfer systems to modulate target RNA levels. Curr Opin Biotechnol 9, 486-496.
Wei Wen Su (2005). Microbial Cell Factories 2005, 4:12 doi:10.1186/1475-2859-4-12.
Yahalom, A., Epel, B.L., Glinka, Z., Macdonald, I.R., and Gordon, D.C. (1987). A Kinetic-Analysis of Phytochrome Controlled Mesocotyl Growth in Zea- Mays Seedlings. Plant Physiol. 84, 390-394.
Yahalom, A., Warmbrodt, R.D., Laird, D.W., Traub, O., Revel, J.P., Willecke, K., and Epel, B.L. (1991). Maize mesocotyl plasmodesmata proteins cross- react with connexin gap junction protein antibodies. Plant Cell 3, 407-417.
Zambryski, P., and Crawford, K. (2000). Plasmodesmata: gatekeepers for cell-to- cell transport of developmental signals in plants. Annu Rev Cell Dev Biol 16, 393-421.
Zhang, H.M., Yang, H., Rech, E.L., Golds, T.J., Davis, A.S., Mulligan, B.J., Cocking, E.C. and Davey, M.R. 1988. Transgenic rice plants produced by electroporation-mediated plasmid uptake into protoplasts. Plant Cell Rep. 7: 379-384.
Zhao, G.R., and Liu, J.Y. (2002). Isolation of a cotton RGP gene: a homolog of reversibly glycosylated polypeptide highly expressed during fiber development. Biochimica Et Biophysica Acta-Gene Structure and Expression 1574, 370-374.

## Claims

1. A method of decreasing the ability of plasmodesmata to transport molecules or viruses in a plant tissue comprising, introducing into cells of the plant tissue the nucleic acid construct encoding a reversibly glycosylated polypeptide pfam 03214, thereby decreasing the ability of plasmodesmata to transport molecules or viruses in the plant tissue.

2. A method of generating plant tissue resistant to spread of a plant virus comprising, introducing into cells of the plant tissue the nucleic acid construct encoding a reversibly glycosylated polypeptide pfam 03214 thereby generating the plant tissue resistant to spread of the plant virus.

3. The method of claim 1 or 2, wherein said reversibly glycosylated polypeptide is encoded by a sequence selected from the group consisting of SEQ ID NOs: 8, 13 and 25-28.

4. The method of claim 1 or 2, wherein said reversibly Glycosylated polypeptide pfam 03214 comprises amino acids 270-end of SEQ ID NOS: 19-24.

## Patentansprüche

1. Ein Verfahren zur Verringerung der Fähigkeit von Plasmodesmen, Moleküle oder Viren in einem Pflanzengewebe zu transportieren, umfassend das Einbringen des Nukleinsäurekonstrukts, das ein reversibel glykosyliertes Polypeptid Pfam 03214 kodiert, in Zellen des Pflanzengewebes, wodurch die Fähigkeit von Plasmodesmen, Moleküle oder Viren in dem Pflanzengewebe zu transportieren, verringert wird.

2. Ein Verfahren zur Erzeugung von Pflanzengewebe, das gegen die Ausbreitung eines Pflanzenvirus resistent ist, umfassend das Einbringen des Nukleinsäurekonstrukts, das ein reversibel glykosyliertes Polypeptid Pfam 03214 kodiert, in Zellen des Pflanzengewebes, wodurch das Pflanzengewebe gegen die Ausbreitung des Pflanzenvirus resistent gemacht wird.

3. Das Verfahren nach Anspruch 1 oder 2, wobei das reversibel glykosylierte Polypeptid von einer Sequenz kodiert wird, ausgewählt aus der Gruppe, bestehend aus den Sequenz-ID-Nummern 8, 13 und 25-28.

4. Das Verfahren nach Anspruch 1 oder 2, wobei das reversibel glykosylierte Polypeptid Pfam 03214 Aminosäuren 270 bis Ende der Sequenz-ID-Nummern 19-24 umfasst.

## Revendications

1. Procédé de diminution de la capacité manifestée par des plasmodesmes à transporter des molécules ou des virus dans un tissu végétal, comprenant le fait d'introduire dans des cellules du tissu végétal le produit de recombinaison d'acide nucléique encodant un polypeptide glycosylé de manière réversible, pfam 03214, pour ainsi diminuer la capacité manifestée par des plasmodesmes à transporter des molécules ou des virus dans le tissu végétal.

2. Procédé de génération de tissu végétal résistant à la propagation d'un virus végétal comprenant le fait d'introduire dans des cellules du tissu végétal le produit de recombinaison d'acide nucléique encodant un polypeptide glycosylé de manière réversible, pfam 03214, pour ainsi générer le tissu végétal résistant à la propagation du virus végétal.

3. Procédé selon la revendication 1 ou 2, dans lequel ledit polypeptide glycosylé de manière réversible est encodé par une séquence choisie parmi le groupe constitué par les SEQ ID NO: 8, 13 et 25-28.

4. Procédé selon la revendication 1 ou 2, dans lequel ledit polypeptide glycosylé de manière réversible, pfam 03214, comprend les acides aminés de 270 à la fin des SEQ ID NO: 19-24.


### SEQUENCE LISTING

<110> Epel, Bernard L. Sagi, Guy L.
<120> CONSTRUCTS AND METHODS FOR GENERATING PLANTS EXHIBITING ALTERED PLASMODESMATAL CONDUCTANCE
<130> 31956
<160> 35
<170> PatentIn version 3.3
<210> 1
   <211> 20
   <212> DNA
   <213> Artificial sequence
<220>
   <223> Single strand DNA oligonucleotide
<220>
   <221> misc_feature
   <222> (9).. (9)
   <223> n is a, c, g, or t
<400> 1
   ttyttycanc cwtaycayct 20
<210> 2
   <211> 20
   <212> DNA
   <213> Artificial sequence
<220>
   <223> Single strand DNA oligonucleotide
<400> 2
   taatacgact cactataggg 20
<210> 3
   <211> 27
   <212> DNA
   <213> Artificial sequence
<220>
   <223> Single strand DNA oligonucleotide
<400> 3
   cgaattcata tggcgggcac ggtgacg 27
<210> 4
   <211> 29
   <212> DNA
   <213> Artificial sequence
<220>
   <223> Single strand DNA oligonucleotide
<400> 4
   ccggatccta ctttggcctt gccatttgc 29
<210> 5
   <211> 5711
   <<12> DNA
   <213> Artificial sequence
<220>
   <223> pET-16 N' His-tag plasmid
<400> 5
   ttctcatgtt tgacagctta tcatcgataa gctttaatgc ggtagtttat cacagttaaa    60
   ttgctaacgc agtcaggcac cgtgtatgaa atctaacaat gcgctcatcg tcatcctcgg   120
   caccgtcacc ctggatgctg taggcatagg cttggttatg ccggtactgc cgggcctctt   180
   gcgggatatc cggatatagt tcctcctttc agcaaaaaac ccctcaagac ccgtttagag   240
   gccccaaggg gttatgctag ttattgctca gcggtggcag cagccaactc agcttccttt   300
   cgggctttgt tagcagccgg atcctcgagc atatgacgac cttcgatatg gccgctgctg   360
   tgatgatgat gatgatgatg atgatgatgg cccatggtat atctccttct taaagttaaa   420
   caaaattatt tctagagggg aattgttatc cgctcacaat tcccctatag tgagtcgtat   480
   taatttcgcg ggatcgagat ctcgatcctc tacgccggac gcatcgtggc cggcatcacc   540
   ggcgccacag gtgcggttgc tggcgcctat atcgccgaca tcaccgatgg ggaagatcgg   600
   gctcgccact tcgggctcat gagcgcttgt ttcggcgtgg gtatggtggc aggccccgtg   660
   gccgggggac tgttgggcgc catctccttg catgcaccat tccttgcggc ggcggtgctc   720
   aacggcctca acctactact gggctgcttc ctaatgcagg agtcgcataa gggagagcgt   780
   cgagatcccg gacaccatcg aatggcgcaa aacctttcgc ggtatggcat gatagcgccc   840
   ggaagagagt caattcaggg tggtgaatgt gaaaccagta acgttatacg atgtcgcaga   900
   gtatgccggt gtctcttatc agaccgtttc ccgcgtggtg aaccaggcca gccacgtttc   960
   tgcgaaaacg cgggaaaaag tggaagcggc gatggcggag ctgaattaca ttcccaaccg  1020
   cgtggcacaa caactggcgg gcaaacagtc gttgctgatt ggcgttgcca cctccagtct  1080
   ggccctgcac gcgccgtcgc aaattgtcgc ggcgattaaa tctcgcgccg atcaactggg  1140
   tgccagcgtg gtggtgtcga tggtagaacg aagcggcgtc gaagcctgta aagcggcggt  1200
   gcacaatctt ctcgcgcaac gcgtcagtgg gctgatcatt aactatccgc tggatgacca  1260
   ggatgccatt gctgtggaag ctgcctgcac taatgttccg gcgttatttc ttgatgtctc  1320
   tgaccagaca cccatcaaca gtattatttt ctcccatgaa gacggtacgc gactgggcgt  1380
   ggagcatctg gtcgcattgg gtcaccagca aatcgcgctg ttagcgggcc cattaagttc  1440
   tgtctcggcg cgtctgcgtc tggctggctg gcataaatat ctcactcgca atcaaattca  1500
   gccgatagcg gaacgggaag gcgactggag tgccatgtcc ggttttcaac aaaccatgca  1560
   aatgctgaat gagggcatcg ttcccactgc gatgctggtt gccaacgatc agatggcgct  1620
   gggcgcaatg cgcgccatta ccgagtccgg gctgcgcgtt ggtgcggata tctcggtagt  1680
   gggatacgac gataccgaag acagctcatg ttatatcccg ccgttaacca ccatcaaaca  1740
   ggattttcgc ctgctggggc aaaccagcgt ggaccgcttg ctgcaactct ctcagggcca  1800
   ggcggtgaag ggcaatcagc tgttgcccgt ctcactggtg aaaagaaaaa ccaccctggc  1860
   gcccaatacg caaaccgcct ctccccgcgc gttggccgat tcattaatgc agctggcacg  1920
   acaggtttcc cgactggaaa gcgggcagtg agcgcaacgc aattaatgta agttagctca  1980
   ctcattaggc accgggatct cgaccgatgc ccttgagagc cttcaaccca gtcagctcct  2040
   tccggtgggc gcggggcatg actatcgtcg ccgcacttat gactgtcttc tttatcatgc  2100
   aactcgtagg acaggtgccg gcagcgctct gggtcatttt cggcgaggac cgctttcgct  2160
   ggagcgcgac gatgatcggc ctgtcgcttg cggtattcgg aatcttgcac gccctcgctc  2220
   aagccttcgt cactggtccc gccaccaaac gtttcggcga gaagcaggcc attatcgccg 2280
   gcatggcggc cgacgcgctg ggctacgtct tgctggcgtt cgcgacgcga ggctggatgg 2340
   ccttccccat tatgattctt ctcgcttccg gcggcatcgg gatgcccgcg ttgcaggcca 2400
   tgctgtccag gcaggtagat gacgaccatc agggacagct tcaaggatcg ctcgcggctc 2460
   ttaccagcct aacttcgatc actggaccgc tgatcgtcac ggcgatttat gccgcctcgg 2520
   cgagcacatg gaacgggttg gcatggattg taggcgccgc cctatacctt gtctgcctcc 2580
   ccgcgttgcg tcgcggtgca tggagccggg ccacctcgac ctgaatggaa gccggcggca 2640
   cctcgctaac ggattcacca ctccaagaat tggagccaat caattcttgc ggagaactgt 2700
   gaatgcgcaa accaaccctt ggcagaacat atccatcgcg tccgccatct ccagcagccg 2760
   cacgcggcgc atctcgggca gcgttgggtc ctggccacgg gtgcgcatga tcgtgctcct 2820
   gtcgttgagg acccggctag gctggcgggg ttgccttact ggttagcaga atgaatcacc 2880
   gatacgcgag cgaacgtgaa gcgactgctg ctgcaaaacg tctgcgacct gagcaacaac 2940
   atgaatggtc ttcggtttcc gtgtttcgta aagtctggaa acgcggaagt cagcgccctg 3000
   caccattatg ttccggatct gcatcgcagg atgctgctgg ctaccctgtg gaacacctac 3060
   atctgtatta acgaagcgct ggcattgacc ctgagtgatt tttctctggt cccgccgcat 3120
   ccataccgcc agttgtttac cctcacaacg ttccagtaac cgggcatgtt catcatcagt 3180
   aacccgtatc gtgagcatcc tctctcgttt catcggtatc attaccccca tgaacagaaa 3240
   tcccccttac acggaggcat cagtgaccaa acaggaaaaa accgccctta acatggcccg 3300
   ctttatcaga agccagacat taacgcttct ggagaaactc aacgagctgg acgcggatga 3360
   acaggcagac atctgtgaat cgcttcacga ccacgctgat gagctttacc gcagctgcct 3420
   cgcgcgtttc ggtgatgacg gtgaaaacct ctgacacatg cagctcccgg agacggtcac 3480
   agcttgtctg taagcggatg ccgggagcag acaagcccgt cagggcgcgt cagcgggtgt 3540
   tggcgggtgt cggggcgcag ccatgaccca gtcacgtagc gatagcggag tgtatactgg 3600
   cttaactatg cggcatcaga gcagattgta ctgagagtgc accatatatg cggtgtgaaa 3660
   taccgcacag atgcgtaagg agaaaatacc gcatcaggcg ctcttccgct tcctcgctca 3720
   ctgactcgct gcgctcggtc gttcggctgc ggcgagcggt atcagctcac tcaaaggcgg 3780
   taatacggtt atccacagaa tcaggggata acgcaggaaa gaacatgtga gcaaaaggcc 3840
   agcaaaaggc caggaaccgt aaaaaggccg cgttgctggc gtttttccat aggctccgcc 3900
   cccctgacga gcatcacaaa aatcgacgct caagtcagag gtggcgaaac ccgacaggac 3960
   tataaagata ccaggcgttt ccccctggaa gctccctcgt gcgctctcct gttccgaccc 4020
   tgccgcttac cggatacctg tccgcctttc tcccttcggg aagcgtggcg ctttctcata 4080
   gctcacgctg taggtatctc agttcggtgt aggtcgttcg ctccaagctg ggctgtgtgc 4140
   acgaaccccc cgttcagccc gaccgctgcg ccttatccgg taactatcgt cttgagtcca 4200
   acccggtaag acacgactta tcgccactgg cagcagccac tggtaacagg attagcagag 4260
   cgaggtatgt aggcggtgct acagagttct tgaagtggtg gcctaactac ggctacacta 4320
   gaaggacagt atttggtatc tgcgctctgc tgaagccagt taccttcgga aaaagagttg 4380
   gtagctcttg atccggcaaa caaaccaccg ctggtagcgg tggttttttt gtttgcaagc 4440
   agcagattac gcgcagaaaa aaaggatctc aagaagatcc tttgatcttt tctacggggt 4500
   ctgacgctca gtggaacgaa aactcacgtt aagggatttt ggtcatgaga ttatcaaaaa 4560
   ggatcttcac ctagatcctt ttaaattaaa aatgaagttt taaatcaatc taaagtatat 4620
   atgagtaaac ttggtctgac agttaccaat gcttaatcag tgaggcacct atctcagcga 4680
   tctgtctatt tcgttcatcc atagttgcct gactccccgt cgtgtagata actacgatac 4740
   gggagggctt accatctggc cccagtgctg caatgatacc gcgagaccca cgctcaccgg 4800
   ctccagattt atcagcaata aaccagccag ccggaagggc cgagcgcaga agtggtcctg 4860
   caactttatc cgcctccatc cagtctatta attgttgccg ggaagctaga gtaagtagtt 4920
   cgccagttaa tagtttgcgc aacgttgttg ccattgctgc aggcatcgtg gtgtcacgct 4980
   cgtcgtttgg tatggcttca ttcagctccg gttcccaacg atcaaggcga gttacatgat 5040
   cccccatgtt gtgcaaaaaa gcggttagct ccttcggtcc tccgatcgtt gtcagaagta 5100
   agttggccgc agtgttatca ctcatggtta tggcagcact gcataattct cttactgtca 5160
   tgccatccgt aagatgcttt tctgtgactg gtgagtactc aaccaagtca ttctgagaat 5220
   agtgtatgcg gcgaccgagt tgctcttgcc cggcgtcaac acgggataat accgcgccac 5280
   atagcagaac tttaaaagtg ctcatcattg gaaaacgttc ttcggggcga aaactctcaa 5340
   ggatcttacc gctgttgaga tccagttcga tgtaacccac tcgtgcaccc aactgatctt 5400
   cagcatcttt tactttcacc agcgtttctg ggtgagcaaa aacaggaagg caaaatgccg 5460
   caaaaaaggg aataagggcg acacggaaat gttgaatact catactcttc ctttttcaat 5520
   attattgaag catttatcag ggttattgtc tcatgagcgg atacatattt gaatgtattt 5580
   agaaaaataa acaaataggg gttccgcgca catttccccg aaaagtgcca cctgacgtct 5640
   aagaaaccat tattatcatg acattaacct ataaaaatag gcgtatcacg aggccctttc 5700
   gtcttcaaga a 5711
<210> 6
   <211> 371
   <212> DNA
   <213> Artificial sequence
<220>
   <223> Cauliflower mosaic virus 35S promoter
<400> 6
   ctcagaagac cagagggcta ttgagacttt tcaacaaagg gtaatatcgg gaaacctcct 60
   cggattccat tgcccagcta tctgtcactt catcgaaagg acagtagaaa aggaaggtgg 120
   ctcctacaaa tgccatcatt gcgataaagg aaaggctatc gttcaagatg cctctaccga 180
   cagtggtccc aaagatggac ccccacccac gaggaacatc gtggaaaaag aagacgttcc 240
   aaccacgtct tcaaagcaag tggattgatg tgatatctcc actgacgtaa gggatgacgc 300
   acaatcccac tatccttcgc aagacccttc ctctatataa ggaagttcat ttcatttgga 360
   gaggacaggc t 371
<210> 7
   <211> 258
   <212> DNA
   <213> Artificial sequence
<220>
   <223> Nitric oxide synthase transcriptional terminator
<400> 7
   ctgcagatcg ttcaaacatt tggcaataaa gtttcttaag attgaatcct gttgccggtc     60
   ttgcgatgat tatcatataa tttctgttga attacgttaa gcatgtaata attaacatgt    120
   aatgcatgac gttatttatg agatgggttt ttatgattag agtcccgcaa ttatacattt    180
   aatacgcgat agaaaacaaa atatagcgcg caaactagga taaattatcg cgcgcggtgt    240
   catctatgtt actagatc                                                  258
<210> 8
   <211> 1401
   <212> DNA
   <213> Arabidopsis thaliana
<400> 8
   gacccacgcg tccggtaaac catggttgag ccggcgaata ctgttggtct tccggtgaac     60
   ccgactccgt tgctgaaaga tgagctcgat atcgtgattc cgactatcag aaacctcgat    120
   ttcctcgaga tgtggaggcc ttttcttcag ccttaccatc tgatcatcgt ccaggacgga    180
   gatccatcga agaagatcca tgtccctgaa ggttacgact acgagctcta caacaggaac    240
   gacattaacc gaatcctcgg acctaaggct tcttgtatct cgtttaagga ttctgcttgt    300
   cgatgctttg ggtacatggt gtctaagaag aagtatatct tcaccattga tgacgattgc    360
   ttcgttgcca aggatccatc aggcaaagca gtgaacgctc ttgagcaaca catcaagaac    420
   cttctctgcc catcgtctcc ctttttcttc aacaccttgt atgatcctta ccgtgaaggt    480
   gctgatttcg tccgtggata ccctttcagt ctccgtgaag gtgtttccac tgctgtttcc    540
   catggtcttt ggctcaacat ccctgactac gatgccccga cccaactcgt gaagcctaag    600
   gagaggaaca ccaggtatgt ggatgctgtc atgaccaacc caaagggaac acttttccca    660
   atgtgtggta tgaacttggc ttttgaccgt gatttgattg gcccggctat gtactttgtt    720
   ctcatgggtg atggtcagcc tattggtcgt tacgacgata tgtgggctgg ttggtgcatc    780
   aaggtgatct gtgaccactt gagcttggga gtgaagaccg gtttaccgta tatctaccac    840
   agcaaagcga gcaacccttt tgttaacctg aagaaggaat acaagggaat cttctggcag    900
   gaggagatca ttccgttctt ccagaacgca aagctatcga aagaagcagt aactgttcag    960
   caatgctaca ttgagctctc aaagatggtc aaggagaagt tgagctcctt agacccgtac   1020
   tttgacaagc ttgcagatgc catggttaca tggattgaag cttgggatga gcttaaccca   1080
   ccagcagcca gtggcaaaag cttgagagca gtatgagcca aaaagaaaaa gccaccaaag   1140
   ttttggttat ttttagctca aattatcgtt acttttaaat ttctgatttt acgaaccttt   1200
   cttgcttttt ttacacattt gagtagtttt catcatcagt actttctcat tgtccggtta   1260
   tggtttttgc atttggttta aatatcaccg gtttatttat aaacagtggt ggattagtag   1320
   tactattttc tgagtttttt tctttgtttc attaataaaa aggccttttc ataggtgttt   1380
   gcaattaaaa aaaaaaaaaa a                                             1401
<210> 9
   <211> 32
   <212> DNA
   <213> Artificial sequence
<220>
   <223> Single strand DNA oligonucleotide
<400> 9
   tctagaattc atggttgagc cggcgaatac tg 32
<210> 10
   <211> 31
   <212> DNA
   <213> Artificial sequence
<220>
   <223> Single strand DNA oligonucleotide
<400> 10
   tctagatact gctctcaagc ttttgccact g 31
<210> 11
   <211> 34
   <212> DNA
   <213> Artificial sequence
<220>
   <223> Single strand DNA oligonucleotide
<400> 11
   tctagaattc atggcgcaat tgtattcttc cgtg 34
<210> 12
   <211> 30
   <212> DNA
   <213> Artificial sequence
<220>
   <223> Single strand DNA oligonucleotide
<400> 12
   tctagaattt ttgccttttg gtgcctcagc 30
<210> 13
   <211> 1393
   <212> DNA
   <213> Arabidopsis thaliana
<400> 13
   gacccacgcg tccgctcaaa tctccacaat cttttctctc caaattctct tctctctgaa     60
   tcaatccaat catggttgag ccggcgaaca ccgttggaat tccggtgaac cacatcccat    120
   tgttgaagga tgagctcgat atcgtgatcc ccacgatccg taacctcgat ttcctggaga    180
   tgtggaggcc tttttttcag ccttaccatc tgattatcgt ccaagatgga gatccatcga    240
   agaccattgc tgtccctgaa gggttcgatt acgaactcta caacaggaac gacatcaacc    300
   gtatccttgg tcctaaagct tcctgcattt ccttcaagga ctctgcttgt cgttgcttcg    360
   gctacatggt ctccaagaag aagtacatct tcactattga tgacgattgc ttcgttgcta    420
   aggatccatc tggaaaagct gtgaacgctc ttgagcaaca catcaagaac cttctctgcc    480
   catcaactcc atttttcttc aacaccttgt acgacccata ccgtgaaggt gctgacttcg    540
   tccgtggata ccctttcagt ctccgtgagg gtgtttccac cgctgtttcc cacggtctgt    600
   ggctcaacat ccctgattac gatgccccta cccaacttgt gaagcctaag gaaaggaaca    660
   caaggtatgt ggatgctgtc atgaccatcc caaaggggac tcttttccct atgtgtggta    720
   tgaacttggc ctatgaccgt gagctcattg gtccggctat gtactttggt ctcatgggtg    780
   atggtcagcc tattggtcgc tacgacgata tgtgggctgg atggtgtatc aaggtgatct    840
   gtgaccattt gggattggga gtgaagacag gtttgcccta catttaccac agcaaagcca    900
   gcaacccgtt tgtgaacttg aagaaggagt acaagggaat cttctggcag gaggatatca    960
   ttcctttctt ccagagccca aagctcacga aagaagctgt gacagttcaa caatgctaca   1020
   tggagctgtc caagttggtg aaggagaagc taagccccat tgatccttac tttgacaagc   1080
   ttgcagatgc tatggtcact tggattgaag cttgggatga gcttaaccca cccactaaag   1140
   cttgagcagc aaaaaaacca ccaccgcagt tttggttatt agctcaacat atcatctatc   1200
   ttctccgttt tgtttttgtt ttgtcttttc tcaaattttc cggcgattct tcagttcgtt   1260
   tttaagttgc cggagtttat ttaaatagtg aatggtgtag ttctatttat gaccgagaca   1320
   atctctcttt tgaggttttt cgtttttgtt tcaataataa aaaatcatat cccttaaaaa   1380
   aaaaaaaaaa aaa                                                      1393
<210> 14
   <211> 31
   <212> DNA
   <213> Artificial sequence
<220>
   <223> Single strand DNA oligonucleotide
<400> 14
   cctaggaatt catggttgag ccggcgaaca c 31
<210> 15
   <211> 30
   <212> DNA
   <213> Artificial sequence
<220>
   <223> Single strand DNA oligonucleotide
<400> 15
   cctaggagct ttagtgggtg ggttaagctc 30
<210> 16
   <211> 38
   <212> DNA
   <213> Artificial sequence
<220>
   <223> Single strand DNA oligonucleotide
<400> 16
   cctaggaatt catggcgggc tacaacctcg aagctatc 38
<210> 17
   <211> 34
   <212> DNA
   <213> Artificial sequence
<220>
   <223> Single strand DNA oligonucleotide
<400> 17
   cctaggcttg gccttgacat ctttgccatc gctc 34
<210> 18
   <211> 25
   <212> PRT
   <213> Artificial sequence
<220>
   <223> Micro sequencing derived peptide sequence
<400> 18
   Asn Leu Asp Phe Leu Gly Met Trp Arg Pro Phe Phe Gln Pro Tyr His
   1               5                   10                  15
   Leu Ile Ile Val Gln Asp Gly Asp Pro
               20                  25
<210> 19
   <211> 357
   <212> PRT
   <213> Arabidopsis thaliana
<400> 19
   Met Val Glu Pro Ala Asn Thr Val Gly Ile Pro Val Asn His Ile Pro
   1               5                   10                  15
   Leu Leu Lys Asp Glu Leu Asp Ile Val Ile Pro Thr Ile Arg Asn Leu
               20                  25                  30
   Asp Phe Leu Glu Met Trp Arg Pro Phe Phe Gln Pro Tyr His Leu Ile
           35                  40                  45
   Ile Val Gln Asp Gly Asp Pro Ser Lys Thr Ile Ala Val Pro Glu Gly
       50                  55                  60
   Phe Asp Tyr Glu Leu Tyr Asn Arg Asn Asp Ile Asn Arg Ile Leu Gly
   65                  70                  75                  80
   Pro Lys Ala Ser Cys Ile Ser Phe Lys Asp Ser Ala Cys Arg Cys Phe
                   85                  90                  95
   Gly Tyr Met Val Ser Lys Lys Lys Tyr Ile Phe Thr Ile Asp Asp Asp
               100                 105                 110
   Cys Phe Val Ala Lys Asp Pro Ser Gly Lys Ala Val Asn Ala Leu Glu
           115                 120                 125
   Gln His Ile Lys Asn Leu Leu Cys Pro Ser Thr Pro Phe Phe Phe Asn
       130                 135                 140
   Thr Leu Tyr Asp Pro Tyr Arg Glu Gly Ala Asp Phe Val Arg Gly Tyr
   145                 150                 155                 160
   Pro Phe Ser Leu Arg Glu Gly Val Ser Thr Ala Val Ser His Gly Leu
                   165                 170                 175
   Trp Leu Asn Ile Pro Asp Tyr Asp Ala Pro Thr Gln Leu Val Lys Pro
               180                 185                 190
   Lys Glu Arg Asn Thr Arg Tyr Val Asp Ala Val Met Thr Ile Pro Lys
           195                 200                 205
   Gly Thr Leu Phe Pro Met Cys Gly Met Asn Leu Ala Tyr Asp Arg Glu
       210                 215                 220
   Leu Ile Gly Pro Ala Met Tyr Phe Gly Leu Met Gly Asp Gly Gln Pro
   225 230 235 240
   Ile Gly Arg Tyr Asp Asp Met Trp Ala Gly Trp Cys Ile Lys Val Ile
   245 250 255
   Cys Asp His Leu Gly Leu Gly Val Lys Thr Gly Leu Pro Tyr Ile Tyr
   260 265 270
   His Ser Lys Ala Ser Asn Pro Phe Val Asn Leu Lys Lys Glu Tyr Lys
   275 280 285
   Gly Ile Phe Trp Gln Glu Asp Ile Ile Pro Phe Phe Gln Ser Pro Lys
   290 295 300
   Leu Thr Lys Glu Ala Val Thr Val Gln Gln Cys Tyr Met Glu Leu Ser
   305 310 315 320
   Lys Leu Val Lys Glu Lys Leu Ser Pro Ile Asp Pro Tyr Phe Asp Lys
   325 330 335
   Leu Ala Asp Ala Met Val Thr Trp Ile Glu Ala Trp Asp Glu Leu Asn
   340 345 350
   Pro Pro Thr Lys Ala
   355
<210> 20
   <211> 364
   <212> PRT
   <213> Arabidopsis thaliana
<400> 20
   Met Val Glu Pro Ala Asn Thr Val Gly Leu Pro Val Asn Pro Thr Pro
   1               5                   10                  15
   Leu Leu Lys Asp Glu Leu Asp Ile Val Ile Pro Thr Ile Arg Asn Leu
               20                  25                  30
   Asp Phe Leu Glu Met Trp Arg Pro Phe Leu Gln Pro Tyr His Leu Ile
           35                  40                  45
   Ile Val Gln Asp Gly Asp Pro Ser Lys Lys Ile His Val Pro Glu Gly
       50                  55                  60
   Tyr Asp Tyr Glu Leu Tyr Asn Arg Asn Asp Ile Asn Arg Ile Leu Gly
   65                  70                  75                  80
   Pro Lys Ala Ser Cys Ile Ser Phe Lys Asp Ser Ala Cys Arg Cys Phe
                   85                  90                  95
   Gly Tyr Met Val Ser Lys Lys Lys Tyr Ile Phe Thr Ile Asp Asp Asp
               100                 105                 110
   Cys Phe Val Ala Lys Asp Pro Ser Gly Lys Ala Val Asn Ala Leu Glu
           115                 120                 125
   Gln His Ile Lys Asn Leu Leu Cys Pro Ser Ser Pro Phe Phe Phe Asn
       130                 135                 140
   Thr Leu Tyr Asp Pro Tyr Arg Glu Gly Ala Asp Phe Val Arg Gly Tyr
   145                 150                 155                 160
   Pro Phe Ser Leu Arg Glu Gly Val Ser Thr Ala Val Ser His Gly Leu
                   165                 170                 175
   Trp Leu Asn Ile Pro Asp Tyr Asp Ala Pro Thr Gln Leu Val Lys Pro
               180                 185                 190
   Lys Glu Arg Asn Thr Arg Tyr Val Asp Ala Val Met Thr Asn Pro Lys
           195                 200                 205
   Gly Thr Leu Phe Pro Met Cys Gly Met Asn Leu Ala Phe Asp Arg Asp
       210                 215                 220
   Leu Ile Gly Pro Ala Met Tyr Phe Val Leu Met Gly Asp Gly Gln Pro
   225                 230                 235                 240
   Ile Gly Arg Tyr Asp Asp Met Trp Ala Gly Trp Cys Ile Lys Val Ile
                   245                 250                 255
   Cys Asp His Leu Ser Leu Gly Val Lys Thr Gly Leu Pro Tyr Ile Tyr
               260                 265                 270
   His Ser Lys Ala Ser Asn Pro Phe Val Asn Leu Lys Lys Glu Tyr Lys
           275                 280                 285
   Gly Ile Phe Trp Gln Glu Glu Ile Ile Pro Phe Phe Gln Asn Ala Lys
       290                 295                 300
   Leu Ser Lys Glu Ala Val Thr Val Gln Gln Cys Tyr Ile Glu Leu Ser
   305                 310                 315                 320
   Lys Met Val Lys Glu Lys Leu Ser Ser Leu Asp Pro Tyr Phe Asp Lys
                   325                 330                 335
   Leu Ala Asp Ala Met Val Thr Trp Ile Glu Ala Trp Asp Glu Leu Asn
               340                 345                 350
   Pro Pro Ala Ala Ser Gly Lys Ser Leu Arg Ala Val
           355                 360
<210> 21
   <211> 362
   <212> PRT
   <213> Arabidopsis thaliana
<400> 21
   Met Ala Gln Leu Tyr Ser Ser Val Lys Pro Thr Pro Met Leu Lys Asp
   1               5                   10                  15
   Glu Leu Asp Ile Val Ile Pro Thr Ile Arg Asn Leu Asp Phe Leu Glu
               20                  25                  30
   Met Trp Arg Pro Phe Phe Glu Gln Tyr His Leu Ile Ile Val Gln Asp
   35 40 45
   Gly Asp Pro Ser Lys Val Ile Asn Ile Pro Val Gly Phe Asp Tyr Glu
   50 55 60
   Leu Tyr Asn Arg Asn Asp Ile Asn Arg Ile Leu Gly Pro Lys Ala Ser
   65 70 75 80
   Cys Ile Ser Phe Lys Asp Ser Ala Cys Arg Cys Phe Gly Tyr Met Val
   85 90 95
   Ser Lys Lys Lys Tyr Ile Tyr Thr Ile Asp Asp Asp Cys Phe Val Ala
   100 105 110
   Lys Asp Pro Thr Gly Lys Glu Ile Asn Ala Leu Glu Gln His Ile Lys
   115 120 125
   Asn Leu Leu Ser Pro Ser Thr Pro His Phe Phe Asn Thr Leu Tyr Asp
   130 135 140
   Pro Tyr Arg Asp Gly Ala Asp Phe Val Arg Gly Tyr Pro Phe Ser Met
   145 150 155 160
   Arg Glu Gly Ala Ile Thr Ala Val Ser His Gly Leu Trp Leu Asn Ile
   165 170 175
   Pro Asp Tyr Asp Ala Pro Thr Gln Leu Val Lys Pro Leu Glu Lys Asn
   180 185 190
   Ser Arg Tyr Val Asp Ala Val Met Thr Ile Pro Lys Gly Thr Leu Phe
   195 200 205
   Pro Met Cys Gly Met Asn Leu Ala Phe Asp Arg Glu Leu Ile Gly Pro
   210 215 220
   Ala Met Tyr Phe Gly Leu Met Gly Asp Gly Gln Pro Ile Gly Arg Tyr
   225 230 235 240
   Asp Asp Met Trp Ala Gly Trp Cys Val Lys Val Ile Cys Asp His Met
   245 250 255
   Gly Trp Gly Val Lys Thr Gly Leu Pro Tyr Ile Trp His Ser Lys Ala
   260 265 270
   Ser Asn Pro Phe Val Asn Leu Lys Lys Glu Tyr Asn Gly Ile Phe Trp
   275 280 285
   Gln Glu Glu Ala Ile Pro Phe Phe Gln Ser Val Thr Leu Pro Lys Glu
   290 295 300
   Cys Thr Ser Val Gln Gln Cys Tyr Leu Glu Leu Ala Lys Leu Val Arg
   305 310 315 320
   Glu Lys Leu Gly Lys Val Asp Pro Tyr Phe Ile Thr Leu Ala Thr Gly
   325 330 335
   Met Val Thr Trp Ile Glu Ala Trp Glu Glu Leu Asn Ser Ala Glu Gly
   340 345 350
   Thr Glu Ala Glu Ala Pro Lys Gly Lys Asn
   355 360
<210> 22
   <211> 364
   <212> PRT
   <213> Arabidopsis thaliana
<400> 22
   Met Ala Gly Tyr Asn Leu Glu Ala Ile Glu Ala Ala Pro Leu Lys Asp
   1               5                   10                  15
   Asp Leu Asp Ile Val Ile Pro Thr Ile Arg Ser Leu Asp Phe Leu Glu
               20                  25                  30
   Gln Trp Arg Pro Phe Leu His His Tyr His Leu Ile Ile Val Gln Asp
           35                  40                  45
   Gly Asp Pro Ser Ile Lys Ile Arg Val Pro Glu Gly Tyr Asp Tyr Glu
       50                  55                  60
   Leu Tyr Asn Arg Asn Asp Ile Asn Arg Ile Leu Gly Pro Arg Ala Asn
   65                  70                  75                  80
   Cys Ile Ser Tyr Lys Asp Gly Gly Cys Arg Cys Phe Gly Phe Met Val
                   85                  90                  95
   Ser Lys Lys Lys Tyr Ile Tyr Thr Ile Asp Asp Asp Cys Phe Val Ala
               100                 105                 110
   Lys Asp Pro Ser Gly Lys Asp Ile Asn Val Ile Ala Gln His Ile Lys
           115                 120                 125
   Asn Leu Glu Thr Pro Ser Thr Pro His Tyr Phe Asn Thr Leu Tyr Asp
       130                 135                 140
   Pro Phe Arg Asp Gly Thr Asp Phe Val Arg Gly Tyr Pro Phe Ser Leu
   145                 150                 155                 160
   Arg Glu Gly Val Gln Thr Ala Ile Ser His Gly Leu Trp Leu Asn Ile
                   165                 170                 175
   Pro Asp Tyr Asp Ala Pro Thr Gln Leu Val Lys Pro Arg Glu Arg Asn
               180                 185                 190
   Thr Arg Tyr Val Asp Ala Val Met Thr Ile Pro Lys Arg Val Leu Tyr
           195                 200                 205
   Pro Met Cys Gly Met Asn Leu Ala Phe Asn Arg Glu Leu Val Gly Pro
       210                 215                 220
   Ala Met Tyr Phe Gly Leu Met Gly Glu Gly Gln Pro Ile Ser Arg Tyr
   225                 230                 235                 240
   Asp Asp Met Trp Ala Gly Trp Ala Ala Lys Val Val Cys Asp His Leu
                   245                 250                 255
   Gly Phe Gly Val Lys Thr Gly Leu Pro Tyr Leu Trp His Ser Lys Ala
               260                 265                 270
   Ser Asn Pro Phe Val Asn Leu Lys Lys Glu His Lys Gly Leu His Trp
           275                 280                 285
   Gln Glu Asp Met Val Pro Phe Phe Gln Asn Leu Arg Leu Ser Lys Glu
       290                 295                 300
   Ser Asp Thr Ala Ala Lys Cys Tyr Met Glu Ile Ser Asn Met Thr Lys
   305                 310                 315                 320
   Glu Lys Leu Thr Lys Val Asp Pro Tyr Phe Glu Lys Leu Ala Asp Ala
                   325                 330                 335
   Met Val Val Trp Ile Glu Ala Trp Glu Glu Leu Asn Pro Pro Val Lys
               340                 345                 350
   Lys Lys Gln Ser Asp Gly Lys Asp Val Lys Ala Lys
           355                 360
<210> 23
   <211> 364
   <212> PRT
   <213> Pisum sativum
<400> 23
   Met Ala Ser Leu Pro Lys Pro Thr Pro Leu Leu Lys Asp Glu Leu Asp
   1               5                   10                  15
   Ile Val Ile Pro Thr Ile Arg Asn Leu Asp Phe Leu Glu Met Trp Arg
               20                  25                  30
   Pro Phe Phe Glu Gln Tyr His Leu Ile Ile Val Gln Asp Gly Asp Pro
           35                  40                  45
   Ser Lys Val Ile Lys Val Pro Glu Gly Phe Asp Tyr Glu Leu Tyr Asn
       50                  55                  60
   Arg Asn Asp Ile Asn Arg Ile Leu Gly Pro Lys Ala Ser Cys Ile Ser
   65                  70                  75                  80
   Phe Lys Asp Ser Ala Cys Arg Cys Phe Gly Tyr Met Val Ser Lys Lys
                   85                  90                  95
   Lys Tyr Ile Tyr Thr Ile Asp Asp Asp Cys Phe Val Ala Lys Asp Pro
               100                 105                 110
   Thr Gly His Glu Ile Asn Ala Leu Glu Gln His Ile Lys Asn Leu Leu
           115                 120                 125
   Ser Pro Ser Thr Pro Phe Phe Phe Asn Thr Leu Tyr Asp Pro Tyr Arg
       130                 135                 140
   Glu Gly Thr Asp Phe Val Arg Gly Tyr Pro Phe Ser Leu Arg Glu Gly
   145                 150                 155                 160
   Val Pro Thr Ala Val Ser His Gly Leu Trp Leu Asn Ile Pro Asp Tyr
                   165                 170                 175
   Asp Ala Pro Thr Gln Leu Val Lys Pro His Glu Arg Asn Thr Arg Phe
               180                 185                 190
   Val Asp Ala Val Leu Thr Ile Pro Lys Gly Ser Leu Phe Pro Met Cys
           195                 200                 205
   Gly Met Asn Leu Ala Phe Asn Arg Glu Leu Ile Gly Pro Ala Met Tyr
       210                 215                 220
   Phe Gly Leu Met Gly Asp Gly Gln Pro Ile Gly Arg Tyr Asp Asp Met
   225                 230                 235                 240
   Trp Ala Gly Trp Cys Ile Lys Val Ile Cys Asp His Leu Gly Tyr Gly
                   245                 250                 255
   Val Lys Thr Gly Leu Pro Tyr Ile Trp His Ser Lys Ala Ser Asn Pro
               260                 265                 270
   Phe Val Asn Leu Lys Lys Glu Tyr Lys Gly Ile Phe Trp Gln Glu Glu
           275                 280                 285
   Ile Ile Pro Phe Phe Gln Ala Ala Thr Leu Ser Lys Asp Cys Thr Ser
       290                 295                 300
   Val Gln Lys Cys Tyr Ile Glu Leu Ser Lys Gln Val Lys Glu Lys Leu
   305                 310                 315                 320
   Gly Thr Ile Asp Pro Tyr Phe Ile Lys Leu Ala Asp Ala Met Val Thr
                   325                 330                 335
   Trp Val Glu Ala Trp Asp Glu Ile Asn Asn Asn Lys Ser Glu Glu Thr
               340                 345                 350
   Thr Ser Thr Lys Ala Ser Glu Val Ala Ala Thr Lys
           355                 360
<210> 24
   <211> 364
   <212> PRT
   <213> Zea mays
<400> 24
   Met Ala Gly Thr Val Thr Val Pro Gly Ser Ser Thr Pro Ser Thr Pro
   1               5                   10                  15
   Leu Leu Lys Asp Glu Leu Asp Ile Val Ile Pro Thr Ile Arg Asn Leu
               20                  25                  30
   Asp Phe Leu Glu Met Trp Arg Ala Phe Phe Gln Pro Tyr His Leu Ile
           35                  40                  45
   Ile Val Gln Asp Gly Asp Pro Thr Lys Thr Ile Lys Val Pro Glu Gly
       50                  55                  60
   Phe Asp Tyr Glu Leu Tyr Asn Arg Asn Asp Ile Asn Arg Ile Leu Gly
   65                  70                  75                  80
   Pro Lys Ala Ser Cys Ile Ser Phe Lys Asp Ser Ala Cys Arg Cys Phe
                   85                  90                  95
   Gly Tyr Met Val Ser Lys Lys Lys Tyr Ile Tyr Thr Ile Asp Asp Asp
               100                 105                 110
   Cys Phe Val Ala Lys Asp Pro Ser Gly Lys Asp Ile Asn Ala Leu Glu
           115                 120                 125
   Gln His Ile Lys Asn Leu Leu Ser Pro Ser Thr Pro Phe Phe Phe Asn
       130                 135                 140
   Thr Leu Tyr Asp Pro Tyr Arg Glu Gly Ala Asp Phe Val Arg Gly Tyr
   145                 150                 155                 160
   Pro Phe Ser Leu Arg Glu Gly Ala His Thr Ala Val Ser His Gly Leu
                   165                 170                 175
   Trp Leu Asn Ile Pro Asp Tyr Asp Ala Pro Thr Gln Leu Val Lys Pro
               180                 185                 190
   Lys Glu Arg Asn Glu Arg Tyr Val Asp Ala Val Met Thr Ile Pro Lys
           195                 200                 205
   Gly Thr Leu Phe Pro Met Cys Gly Met Asn Leu Ala Phe Asp Arg Asp
       210                 215                 220
   Leu Ile Gly Pro Ala Met Tyr Phe Gly Leu Met Gly Asp Gly Gln Pro
   225                 230                 235                 240
   Ile Gly Arg Tyr Asp Asp Met Trp Ala Gly Trp Cys Val Lys Val Ile
                   245                 250                 255
   Cys Asp His Leu Ser Leu Gly Val Lys Thr Gly Leu Pro Tyr Ile Trp
               260                 265                 270
   His Ser Lys Ala Ser Asn Pro Phe Val Asn Leu Lys Lys Glu Tyr Lys
           275                 280                 285
   Gly Ile Phe Trp Gln Glu Asp Ile Ile Pro Phe Phe Gln Asn Val Thr
       290                 295                 300
   Ile Pro Lys Asp Cys Asp Thr Val Gln Lys Cys Tyr Ile Tyr Leu Ser
   305                 310                 315                 320
   Gly Gln Val Lys Glu Lys Leu Gly Thr Ile Asp Pro Tyr Phe Val Lys
                   325                 330                 335
   Leu Gly Asp Ala Met Val Thr Trp Ile Glu Ala Trp Asp Glu Leu Asn
                   340                 345                 350
   Pro Ser Thr Pro Ala Ala Ala Asn Gly Lys Ala Lys
           355                 360
<210> 25
   <211> 1374
   <212> DNA
   <213> Arabidopsis thaliana
<400> 25
   actctttctt ccttgtcgtt tcaaatggcg caattgtatt cttccgtgaa acccacgcca     60
   atgttgaaag atgagctaga cattgtgata cctacgattc gaaacctcga cttccttgag    120
   atgtggcgac ctttctttga gcagtatcat ttgatcattg ttcaagatgg tgatccttcg    180
   aaagtcatca acattccagt agggtttgac tacgagctct acaacaggaa cgacatcaat    240
   cgaatcttgg gtcccaaggc ctcttgcatt tccttcaagg actctgcttg tcgttgcttc    300
   ggttacatgg tctccaaaaa gaagtacatc tacaccattg atgatgattg ctttgttgca    360
   aaggatccaa cagggaaaga gatcaatgcg cttgagcagc atatcaagaa cctcttatct    420
   ccatcaactc cgcatttctt taacacactt tatgatccat acagagatgg ggcagacttt    480
   gttcgtggat accctttcag tatgcgcgaa ggtgccataa ccgcggtctc tcatggcctc    540
   tggctcaaca ttcctgacta tgatgctccc actcagcttg tgaaacctct tgagaaaaat    600
   tccaggtatg ttgatgcagt catgaccatt cccaaaggaa ctctttttcc tatgtgtggt    660
   atgaaccttg cttttgaccg tgagctgatt ggtccggcta tgtactttgg gcttatgggt    720
   gatggacagc caattggacg ctatgacgac atgtgggctg gttggtgtgt caaggtgata    780
   tgtgaccaca tgggatgggg agtgaagacg ggtttgcctt acatatggca cagcaaagcc    840
   agcaacccat tcgtgaacct gaaaaaggaa tacaacggaa tattttggca ggaagaggcg    900
   attccgttct ttcaatccgt gactcttcct aaagagtgca cttcggtgca gcaatgctac    960
   ctcgagctgg ccaagctcgt gagagagaaa cttgggaagg ttgatcctta cttcatcaca   1020
   cttgcaactg gaatggtcac ctggatcgaa gcttgggagg agcttaactc cgcggaggga   1080
   actgaggctg aggcaccaaa aggcaaaaat tagtaaagag aaggtcactg aataaggcaa   1140
   atggtattgt ttactaggag aaaagggttt gagtcacatg tgcaaccctc tctgtttttc   1200
   ttaatcataa tttgatttgc ttctgtcttt aatttcttgt catcagtatt tgtgttctca   1260
   gtgcattgtc agtttgtcac aaaaagagaa ggtgtaatgt gctctatgca ttttgttttc   1320
   tttaaggttt ctaaactctt tatctatgaa atatctatca ttcctatttt cctt         1374
<210> 26
   <211> 1251
   <212> DNA
   <213> Arabidopsis thaliana
<400> 26
   taagtaccaa aaatggcggg ctacaacctc gaagctatcg aagcggcacc tttgaaagac     60
   gatctggata ttgtgatccc aaccatcaga agcctcgact ttctagagca atggagacca    120
   tttttgcacc attaccacct gatcattgtc caagatggag acccttccat caagatccga    180
   gttcccgagg gttatgacta cgagctctac aaccgtaatg atatcaacag gatccttggt    240
   ccaagagcta attgtatctc ctacaaagat ggtggttgtc gctgctttgg cttcatggtt    300
   tctaaaaaga agtatatcta caccattgat gatgattgct ttgtggcaaa ggatccaagt    360
   gggaaagaca tcaatgtgat agctcagcac ataaagaacc ttgaaacacc ttcaacacca    420
   cactacttca acactctcta tgaccctttt agagatggaa ctgatttcgt cagagggtat    480
   cctttcagtc taagggaagg tgtccaaacc gccatatctc atggtctttg gctcaacatc    540
   cctgactatg atgccccaac ccaacttgtg aagccccgcg aacgtaacac gaggtatgtg    600
   gatgcagtaa tgacaattcc aaaaagagta ttgtacccaa tgtgtggtat gaatcttgct    660
   ttcaatagag agcttgttgg acctgctatg tactttggac ttatgggtga aggccagccc    720
   atttctcggt acgatgacat gtgggctggt tgggcagcaa aggtggtgtg tgaccacttg    780
   ggctttgggg tcaagactgg tctgccgtat ctgtggcaca gcaaagccag taacccattc    840
   gtgaacctga agaaagagca taagggactc cactggcaag aagatatggt tcctttcttc    900
   caaaaccttc gtctctcgaa agaatctgac actgcagcta aatgttatat ggaaatttca    960
   aatatgacaa aagaaaagct cactaaagtg gatccttact ttgagaagtt ggctgatgca   1020
   atggtggttt ggattgaggc gtgggaggag cttaacccgc cggttaagaa gaagcagagc   1080
   gatggcaaag atgtcaaggc caagtgatga agataatgag tgatcctatt gttgctaatg   1140
   aataagtatg ttgtgagttt atagattata tgatattgta tttattttct ccattttgga   1200
   agtgtgcgtt attatgaaat aatttggcat gtattaagcg tgattggaag a            1251
<210> 27
   <211> 1298
   <212> DNA
   <213> Pisum sativum
<400> 27
   gcacgagcgc acttctcaac aatggcttcg ttacccaaac caactccact cttgaaagac      60
   gaactcgaca tcgtcatccc tacgatccgt aaccttgatt tcctggagat gtggagaccc     120
   ttttttgaac agtaccatct catcattgtt caagatggtg acccttctaa ggttatcaag     180
   gttcctgaag gtttcgatta tgaactgtat aatcggaatg atatcaatag gatcttgggt     240
   cctaaagctt cgtgtatctc cttcaaggat tcggcttgtc gttgctttgg gtatatggtt     300
   tcgaagaaga agtatatcta caccattgat gatgattgct ttgttgctaa agacccaact     360
   gggcatgaaa tcaatgcact tgagcagcac attaagaatc tccttagtcc atccactcca     420
   tttttcttca acacccttta cgatccatac agagaaggta ctgatttcgt ccgtggatac    480
   cctttcagtc ttcgtgaagg tgtccccact gccgtttctc acggcctttg gctcaacata     540
   cctgattacg atgctccaac tcagcttgtc aagccccatg agaggaacac taggtttgtt     600
   gatgctgttc tgaccattcc caaaggaagt ctgttcccca tgtgcggtat gaatctggca     660
   tttaaccgtg aactgattgg acctgcaatg tacttcggac tcatgggtga tggtcagcct     720
   attggacgct acgacgatat gtgggctgga tggtgcataa aggttatctg tgaccatttg     780
   ggatatggag tgaaaaccgg actaccttac atttggcaca gcaaagcaag caacccattt     840
   gttaatctga aaaaggagta caaaggtatc ttctggcaag aagagatcat tccatttttc     900
   caagctgcaa ccctttcaaa agattgcacc tctgttcaga aatgctacat tgaactctcc     960
   aagcaagtca aggagaaact tggaactatt gatccctatt tcatcaaact cgccgatgcc    1020
   atggtcactt gggttgaagc ttgggatgag attaataaca acaaatctga agagacaact    1080
   tcaaccaaag cttctgaggt tgctgctacc aagtgaaaca acttatagtt gatgaggaag    1140
   agagtagttt tcaatcagtt ttattattgt tatcatattt gttagcatta tattatgatt    1200
   cttgttgatt ttgctagatt ccagaacaat ttattgatat ttatgttatt aatatttata    1260
   ttaaaaaaaa aaaaaaaaaa aaaaacctcg aggggggg                             1298
<210> 28
   <211> 1480
   <212> DNA
   <213> Zea mays
<400> 28
   aacctcctct gctctccccc acctcccggt ccggcagcag agagcggggg tgcagagaga     60
   tcgagatccg gaggccgcga gtgggagcgc caccaccagt gtctgcggca atggcgggca    120
   cggtgacggt cccggggtcg tcgaccccct ccacgccgct gctcaaggac gagctcgaca    180
   tcgtgatccc gacgatccgc aacctcgact tcctggagat gtggcgggcc ttcttccagc    240
   cctaccacct catcatcgtg caggacggcg acccgaccaa gaccatcaag gtgcccgagg    300
   gcttcgacta cgagctctac aaccgcaacg acatcaaccg catcctcggg cccaaggcct    360
   cctgtatctc cttcaaggac tccgcctgcc gctgcttcgg ctacatggtc tccaagaaga    420
   agtacatcta caccatcgac gacgactgct tcgttgccaa ggacccatct ggcaaggata    480
   tcaatgctct tgagcagcac atcaagaacc tcctcagccc atccaccccg ttcttcttca    540
   acaccctgta cgacccctac cgtgagggtg ctgactttgt gcgtggatac cccttcagcc    600
   tcagggaggg tgctcacacc gccgtctccc acggcctgtg gctgaacatc cctgactatg    660
   atgctcccac acagctggtc aagcccaagg agaggaatga gaggtatgtt gatgctgtca    720
   tgacaatccc caagggaacc ttgttcccca tgtgtggcat gaaccttgcc ttcgacaggg    780
   atctcattgg ccctgctatg tactttggtc tcatgggtga tggccagccc atcggtcgct    840
   acgacgacat gtgggcagga tggtgtgtga aggtcatctg cgaccacctg agcctgggag    900
   tcaagactgg cctgccatac atctggcaca gcaaggctag caaccccttc gttaacttga    960
   agaaggaata caagggcatc ttctggcagg aggacatcat ccccttcttc cagaacgtga   1020
   ccatccccaa ggactgcgac accgtccaaa agtgctacat ctacctctct ggacaagtga   1080
   aggagaagct gggcacgatt gacccctact ttgtcaagct cggtgacgcc atggtcacct   1140
   ggatcgaggc ctgggatgag ctgaacccgt cgacccctgc cgccgcaaat ggcaaggcca   1200
   agtagatgtg ccatcacttc ggcactggag tgaaaaagaa agtatgattg ggtcctgcct   1260
   aggccccata gatatagctc ttttttgaga aggaagagag attaattcag cagcagcttt   1320
   tataaattct ttgttgtcat gaagcaggtg gttcttttgt agttgatgaa agggatttgt   1380
   cgtcgctatc gtttacataa ttcagacaat aattttatat tgtgtaattc tgagaatcaa   1440
   agcactgttt gtgttatagc aaaaaaaaaa aaaaaaaaaa                         1480
<210> 29
   <211> 729
   <212> DNA
   <213> Artificial sequence
<220>
   <223> sGFP
<400> 29
   atggtgagca agggcgagga gctgttcacc ggggtggtgc ccatcctggt cgagctggac      60
   ggcgacgtaa acggccacaa gttcagcgtg tccggcgagg gcgagggcga tgccacctac     120
   ggcaagctga ccctgaagtt catctgcacc accggcaagc tgcccgtgcc ctggcccacc     180
   ctcgtgacca ccttcaccta cggcgtgcag tgcttcagcc gctaccccga ccacatgaag     240
   cagcacgact tcttcaagtc cgccatgccc gaaggctacg tccaggagcg caccatcttc     300
   ttcaaggacg acggcaacta caagacccgc gccgaggtga agttcgaggg cgacaccctg     360
   gtgaaccgca tcgagctgaa gggcatcgac ttcaaggagg acggcaacat cctggggcac     420
   aagctggagt acaactacaa cagccacaac gtctatatca tggccgacaa gcagaagaac     480
   ggcatcaagg tgaacttcaa gatccgccac aacatcgagg acggcagcgt gcagctcgcc     540
   gaccactacc agcagaacac ccccatcggc gacggccccg tgctgctgcc cgacaaccac     600
   tacctgagca cccagtccgc cctgagcaaa gaccccaacg agaagcgcga tcacatggtc     660
   ctgctggagt tcgtgaccgc cgccgggatc actcacggca tggacgagct gtacaagggg     720
   atcctctag                                                              729
<210> 30
   <211> 685
   <212> DNA
   <213> Artificial sequence
<220>
   <223> DsRed1
<400> 30
   cgccaccatg gtgcgctcct ccaagaacgt catcaaggag ttcatgcgct tcaaggtgcg      60
   catggagggc accgtgaacg gccacgagtt cgagatcgag ggcgagggcg agggccgccc     120
   ctacgagggc cacaacaccg tgaagctgaa ggtgaccaag ggcggccccc tgcccttcgc     180
   ctgggacatc ctgtcccccc agttccagta cggctccaag gtgtacgtga agcaccccgc     240
   cgacatcccc gactacaaga agctgtcctt ccccgagggc ttcaagtggg agcgcgtgat     300
   gaacttcgag gacggcggcg tggtgaccgt gacccaggac tcctccctgc aggacggctg     360
   cttcatctac aaggtgaagt tcatcggcgt gaacttcccc tccgacggcc ccgtaatgca     420
   gaagaagacc atgggctggg aggcctccac cgagcgcctg tacccccgcg acggcgtgct     480
   gaagggcgag atccacaagg ccctgaagct gaaggacggc ggccactacc tggtggagtt     540
   caagtccatc tacatggcca agaagcccgt gcagctgccc ggctactact acgtggactc     600
   caagctggac atcacctccc acaacgagga ctacaccatc gtggagcagt acgagcgcac     660
   cgagggccgc caccacctgt tcctg                                            685
<210> 31
   <211> 727
   <212> DNA
   <213> Artificial sequence
<220>
   <223> ECFP
<400> 31
   cgccaccatg gtgagcaagg gcgaggagct gttcaccggg gtggtgccca tcctggtcga     60
   gctggacggc gacgtaaacg gccacaagtt cagcgtgtcc ggcgagggcg agggcgatgc    120
   cacctacggc aagctgaccc tgaagttcat ctgcaccacc ggcaagctgc ccgtgccctg    180
   gcccaccctc gtgaccaccc tgacctgggg cgtgcagtgc ttcagccgct accccgacca    240
   catgaagcag cacgacttct tcaagtccgc catgcccgaa ggctacgtcc aggagcgcac    300
   catcttcttc aaggacgacg gcaactacaa gacccgcgcc gaggtgaagt tcgagggcga    360
   caccctggtg aaccgcatcg agctgaaggg catcgacttc aaggaggacg gcaacatcct    420
   ggggcacaag ctggagtaca actacatcag ccacaacgtc tatatcaccg ccgacaagca    480
   gaagaacggc atcaaggcca acttcaagat ccgccacaac atcgaggacg gcagcgtgca    540
   gctcgccgac cactaccagc agaacacccc catcggcgac ggccccgtgc tgctgcccga    600
   caaccactac ctgagcaccc agtccgccct gagcaaagac cccaacgaga agcgcgatca    660
   catggtcctg ctggagttcg tgaccgccgc cgggatcact ctcggcatgg acgagctgta    720
   caagtaa                                                              727
<210> 32
   <211> 21
   <212> DNA
   <213> Artificial sequence
<220>
   <223> SiRNA oligonucleotide
<400> 32
   ucgauaucgu gauuccgact a 21
<210> 33
   <211> 21
   <212> DNA
   <213> Artificial sequence
<220>
   <223> SiRNA oligonucleotide
<400> 33
   gaagaccggu uuaccguaua t 21
<210> 34
   <211> 21
   <212> DNA
   <213> Artificial sequence
<220>
   <223> Antisense oligonucleotide
<220>
   <221> misc_feature
   <223> Oligo has a phosphorothioate backbone
<400> 34
   cggtcttcac tcccaagctc a 21
<210> 35
   <211> 19
   <212> DNA
   <213> Artificial sequence
<220>
   <223> Antisense oligonucleotide
<220>
   <221> misc_feature
   <223> Oligo has a phosphorothioate backbone
<400> 35
   tcccttgtat tccttcttc 19